# EUROPEAN PATENT SPECIFICATION

(11) **EP 3 077 467 B1**
(45) Date of publication and mention of the grant of the patent: **02.11.2022**
(21) Application number: 14816144.1
(22) Date of filing: 03.12.2014
(51) Int. Cl.: C09D 167/08, C09F 9/00

(54) **DRIER COMPOSITION AND USE THEREOF**
TROCKNERZUSAMMENSETZUNG UND VERWENDUNG DAVON
COMPOSITION DE SICCATIF ET SON UTILISATION

(30) Priority: 03.12.2013 WO PCT/EP2013/075430
(43) Date of publication of application: 12.10.2016
(73) Proprietor: PPG Europe B.V., 1422 AD Uithoorn (NL)
(72) Inventor: ANG, Tjian Hong, NL-1333 NA Almere (NL); WEIJNEN, John, NL-2408 KA Alphen aan den Rijn (NL)
(74) Representative: Lesthaeghe, David
(86) International application number: PCT/EP2014/076431
(87) International publication number: WO 2015/082553

(56) References cited:
- EP-A1- 2 474 578
- WO-A1-2013/092441

## Description

### FIELD OF THE INVENTION

The invention relates to a drier system for a coating composition, preferably for an autoxidizable alkyd based coating composition.

### BACKGROUND OF THE INVENTION

Architectural, air-drying coating compositions such as paints, lacquers and varnishes commonly comprise three main components: an autoxidizable binder polymer, which is the film-forming component, a solvent, which is the carrier for the non-volatile components, and driers or siccatives, which influence the drying speed of the composition. Autoxidizable binder polymers can be diluted in an organic solvent such as white spirit or hydrogenated white spirit. Alternatively, autoxidizable binder polymers can be dispersed in water.

Autoxidizable alkyd resins are long-established binder polymers for film-forming coating compositions acknowledged for their esthetic properties, low surface tension (which enables the wetting of and adhesion on a wide variety of substrates and facilitates pigment wetting), applicability by various techniques, and cost-effectiveness. Because of these properties, alkyd resins are the most widely used air drying binders in coating compositions. Autoxidizable alkyd resins comprise drying or semi-drying unsaturated fatty acids or oils, which are generally attached to the polyester backbone of polyols and polycarboxylic acids. When the coating composition is applied to a substrate, the drying process starts by solvent evaporation and the binder polymers undergo autoxidation and subsequently form cross-links between the polymer chains resulting in a solid and coherently dried film. Autoxidation is a free radical chain process that takes place when alkyd resins are exposed to air. Autoxidation is a free radical chain process which can be subdivided into three stages: chain initiation, propagation, and termination. Once free radicals are formed, they react in a chain and convert the unsaturated moieties of alkyd resins in interpolymer crosslinks. The drying process of autoxidizable architectural coating compositions takes place at ambient temperatures ranging from 0 to 40° C, whereby the presence of oxygen is essential. Since the drying process proceeds slowly, the chemical conversion of alkyd resins is habitually catalyzed by salts of metal ions as catalytic oil drying agents. These salts of metal ions act as driers or siccatives.

These metal salts, containing either alkaline metals, alkaline-earth metals, transition metals or rare earth metals, affect the autoxidation of the binder with air and/or catalyze cross-linking of the unsaturated fatty acid chains. The drying time can consequently be reduced from days to hours. The presence of efficient driers is therefore essential for the drying of air-drying coating compositions.

Primary driers based on cobalt are the most widely used because of their good performance at ambient temperature. However, because of the suspected carcinogenicity of cobalt carboxylates it is desired to find alternative drier compounds that show an equal or even better drying performance than that of cobalt driers and can replace cobalt based driers completely in alkyd based oxidatively air-drying coatings. The anticipated imminent reclassification of cobalt metal and cobalt carboxylates as Class 1B carcinogenic materials has prompted the development of cobalt-free metal driers for alkyds. Hence there is an increasing demand for alternative, non-cobalt based driers.

Recently developed curing agents for air-drying alkyd resins based on iron complexes containing tetradentate, pentadentate or hexadentate nitrogen donor ligands (as disclosed in WO2008003652) show a highly efficient drying behavior under ambient conditions, and higher initial whiteness and lower yellowing tendency compared to other technologies. However, these driers suffer from the disadvantage that their catalytic activity is difficult to control leading to untimely skin formation during storage.

A liquid coating composition containing resins based on unsaturated fatty acids and metal driers, when exposed to air, will rapidly convert into a drying polymeric matrix. For the formation of surface skin on air-drying coatings by oxidative polymerization processes oxygen is required. Even in a closed container, in which the ullage (or headspace) between the product surface and the closed lid is sufficiently large, a tough rubber-like skin on the surface of the liquid coating material will develop. Skinning in the paint container during storage results in loss of quality and quantity of the coating product. Removal of the skin is both awkward and time consuming and results in a waste of a substantial amount of paint. Moreover, the concentration of driers found in the skin is disproportionate and removal will lead to a prolongation of drying time of the remaining coating material.

To prevent untimely oxidative drying leading to skinning of the alkyd paint and to improve the stability during storage anti-skinning agents are included in the formulation. Oximes, and in particular methyl ethyl ketoxime (MEKO), are known to reduce skin formation considerably, particularly in alkyd formulations containing cobalt-based driers. Although the mechanism associated with the anti-skinning performance of oximes is still not accepted unanimously, the prevailing hypotheses concerns the association of the oxime to the free coordination sites of the metal carboxylate thereby suppressing the activity of the catalyst. During storage in a closed container the weak metal - oxime complex will stay intact. However, after opening of the can or paint application, the complex will dissociate and the relative volatile oxime will be released into the surrounding atmosphere. Without being bold on the theory, it is supposed that on an iron multidentate ligand complex there are insufficient free coordination sites left for methyl ethyl ketoxime to associate and suppress the catalytic activity.

In WO2012093250 it was shown that MEKO as sole anti-skinning agent in alkyd formulations containing iron-hexadentate ligand complex as drier, was ineffective to prevent skin formation on the surface of the paint formulation during storage. WO2012093250 describes the use of an aqueous solution of iron salts and a polydentate accelerant ligand in an oxidatively curable coating formulation to reduce the skin formation of such as a formulation. However, the example section shows that even this approach does not provide properties similar to a traditional Co-based drier system.

Substances which have been ascribed as anti-skinning agents include substituted phenols, hydroquinones, aliphatic and aromatic amines, tin compounds, azones, α-hydroxyketones, hydroxylamines, β-dicarbonyl compounds, natural antioxidants such as tocopherol and isoascorbates, solvents as dipentene and oximes. These organic substances inhibit the reaction of the drier with atmospheric oxygen by binding the oxygen or by complexing the drier metal.

Especially diethyl hydroxylamine and combinations thereof have been documented for their ability to inhibit or slow the propensity for skinning at the air-alkyd interphase.

DE-A 1 519 103 discloses the use of N,N-dialkylated hydroxylamines such as diethyl hydroxylamine as anti-skinning agents. Diethyl hydroxylamine has found use as an anti-skinning agent. Although diethyl hydroxyl amine is more volatile compared to MEKO, this substance compound binds more strongly to cobalt driers than MEKO and as such does not dissociate at the same rate, which results a poorer drying performance. Attempts have been made to balance this negative effect by the addition of an accelerator to promote the drying process.

US6730157 describes an anti-skinning agents composed of organic hydroxylamines and β-dicarbonyl compounds or organic hydroxylamines and formic acid derivatives.

WO2008127739 describes a non-oxime anti-skinning composition including an organic oxygen scavenger as diethyl hydroxylamine and a drying accelerator as triphenylphosphite and strontium carboxylate.

In US20050272841 anti-skinning agents containing mixtures of organic hydroxylamines as diethyl hydroxylamine and alkyl amines and/or alkyl alkanolamines are disclosed. Combining the alkyl amines and/or alkyl alkanolamines with hydroxylamines should avoid the disadvantages of hydroxylamines alone as severe delays in drying and often also reduction of film hardness values.

WO2007027460 describes anti-skinning agents for oxidatively drying coatings comprising mixtures of organic or inorganic oxygen scavengers as diethyl hydroxylamine and nitrogen-containing aromatic compounds. The nitrogen-containing aromatic compound acts as a drier promoter for the metal drier in the air drying coating.

US2007022910 discloses an anti-skinning agent comprising a synergistic combination of a hydroxylamine as diethyl hydroxylamine and a hydroquinone. Because of the synergistic effect, a reduced oxygen scavenger concentration allows for acceptable through drying.

EP 2 474 578 discloses paint, ink and other coating formulations, particularly alkyd-based formulations comprising metal driers, such as iron- and manganese-containing compounds, which exhibit a reduced propensity to develop a skin on storage.

WO 2013/092441 discloses a process for producing a Mn-based drier for use in an auto-oxidisable coating composition.

The primary drawback to these systems composed of organic hydroxylamines such as diethyl hydroxylamine is that they bind to metal driers more strongly than MEKO and concomitantly do not dissociate at the same rate, resulting is a poorer drying performance. Also the combinations of organic hydroxyl amines and drying accelerators or other substances to promote the drying process and to accelerate the hardness build-up, has not led to acceptable curing times. One of the ambitions of the paint market is to reduce the down time during maintenance work and facilitate fast reoccupation of the residence which makes that anti-skinning materials based on this technology have not been successful because at dosing quantities where adequate anti-skinning performance is achieved, the drying performance is substantially compromised.

US2715072 describes 2-tertairy-alkyl-4-alkoxyphenol as anti-skinning agents in drying oil compositions. However, phenol derivatives tend to cause severe retardation of the drying time and induce undesirable discolorations, which makes the use of these materials impractical in cases where clear varnishes, light colored lacquers, etc., are involved.

US 6224659 discloses the use of tin(II) and tin(IV) carboxylates as anti-skinning agents for oxidatively drying binders. Although chemically showing similarities with metal driers, these tin carboxylates are not catalytically active in the oxidatively drying process but in contrast retard the drying to an objectionable degree.

Thus, there exists a need to improve the drying performance of non-cobalt primary drier compositions while at the same time also improving their anti-skinning properties without the excessive use of additional anti-skinning agents.

### SUMMARY OF THE INVENTION

The inventors have surprisingly found that a specific combination of a selected Fe-drier and a manganese, cerium, vanadium or copper drier with specific ligands reduces the skinning tendency of an alkyd-based coating composition substantially and that additional anti-skinning agent (beside an optional ketoxime) is not needed. Since additional anti-skinning agent is not required, this eliminates drying retardation caused by any additional anti-skinning agent. As a result, the reduced skinning tendencies are accompanied by concomitantly faster drying times.

The present invention provides novel drier combinations suitable for oxidatively air-drying alkyd based coating formulations, which drier combinations show a drying activity at least comparable or superior to cobalt based driers while simultaneously providing reduced skinning tendencies. The present invention additionally provides alkyd-based coating compositions that comprise the aforementioned novel drier combinations.

According to a first aspect, the present invention relates to a drier composition for an autoxidizable alkyd based coating composition, comprising:
a) at least one Fe complex comprising Fe and at least one nitrogen donor ligand, wherein said at least nitrogen donor ligand is selected from the group comprising tridentate, tetradentate, pentadentate and hexadentate nitrogen donor ligands;
b) at least one metal salt of a carboxylic acid, wherein the metal is selected from the group comprising Mn, Ce, V, and Cu; and
c) at least one ligand comprising at least one moiety selected from the group comprising 1,4,7-tri-azacyclononanyl, 2,2'-bipyridyl, 1,10-phenantrolinyl, imidazolyl, pyrazolyl, porphyrinyl, aliphatic, cycloaliphatic, and aromatic amines.

According to a second aspect, the invention also relates to a coating composition, comprising:
a) at least one autoxidizable alkyd based binder; and
b) a drier composition according to the first aspect of the invention.

According to a third aspect, the invention also relates to the use of the coating composition according to the second aspect in a varnish, lacquer, paint, stain, enamel, printing ink or floor covering.

According to a fourth aspect, the invention also relates substrate having applied thereon a coating composition according to the second aspect.

Preferred embodiments of the invention are disclosed in the detailed description and appended claims. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### DETAILED DESCRIPTION OF THE INVENTION

When describing the compositions of the invention, the terms used are to be construed in accordance with the following definitions, unless a context dictates otherwise.

As used in the specification and the appended claims, the singular forms "a", "an," and "the" include both singular and plural referents unless the context clearly dictates otherwise. By way of example, "a binder" means one binder or more than one binder.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

As used herein, the term "and/or," when used in a list of two or more items, means that any one of the listed items can be employed by itself or any combination of two or more of the listed items can be employed. For example, if a list is described as comprising group A, B, and/or C, the list can comprise A alone; B alone; C alone; A and B in combination; A and C in combination, B and C in combination; or A, B, and C in combination.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art.

The term "about" as used herein when referring to a measurable value such as a parameter, an amount, a temporal duration, and the like, indicate that a value includes the standard deviation of error for the device or method being employed to determine the value. Preferably the term "about" is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

As used herein the term "nitrogen-donor ligand" or "ligand" or "L" is an organic structure or molecule which will support coordinating nitrogen atoms. In the present invention, said at least one nitrogen-donor ligand is selected from the group comprising tridentate, tetradentate, pentadentate and hexadentate nitrogen donor ligands.

Whenever the term "substituted" is used herein, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation from a reaction mixture.

Where groups can be substituted, such groups may be substituted with one or more, and preferably one, two or three substituents. Substituents may be selected from but not limited to, for example, the group comprising halogen; C₁₋₆alkyl; haloC₁₋₆alkyl; haloC₁₋₆alkyloxy; hydroxyl, hydroxyC₁₋₆alkyl, amino, aminoC₁₋₆alkyl, and carboxyl.

The term "hydroxyl" or "hydroxy" as used herein refers to the group -OH.

The term "carboxy" or "carboxyl" or "hydroxycarbonyl" as used herein refers to the group -C(=O)OH.

The term "alkyl", as a group or part of a group, refers to a hydrocarbyl group of Formula -CₙH₂ₙ₊₁ wherein n is a number of at least 1. Alkyl groups may be linear, or branched and may be substituted as indicated herein. Generally, the alkyl groups comprise from 1 to 24 carbon atoms, preferably from 1 to 12 carbon atoms, preferably from 1 to 10 carbon atoms, preferably from 1 to 6 carbon atoms, more preferably 1, 2, 3, 4, 5, 6 carbon atoms. When a subscript is used herein following a carbon atom, the subscript refers to the number of carbon atoms that the named group may contain. For example, the term "C₁₋₂₄alkyl", as a group or part of a group, refers to a hydrocarbyl group of Formula -CₙH₂ₙ₊₁ wherein n is a number ranging from 1 to 24. Thus, for example, C₁₋₂₄alkyl groups include all linear, or branched alkyl groups having 1 to 24 carbon atoms, and thus includes for example methyl, ethyl, n-propyl, *i*-propyl, 2-methyl-ethyl, butyl and its isomers (e.g. n-butyl, *i*-butyl and *t*-butyl); pentyl and its isomers, hexyl and its isomers, heptyl and its isomers, octyl and its isomers, nonyl and its isomers, decyl and its isomers, undecyl and its isomers, dodecyl and its isomers, tridecyl and its isomers, tetradecyl and its isomers, pentadecyl and its isomers, hexadecyl and its isomers, heptadecyl and its isomers, octadecyl and its isomers, nonadecyl and its isomers, icosyl and its isomers, and the like. For example, C₁₋₁₂alkyl includes all linear, or branched alkyl groups having 1 to 12 carbon atoms, and thus includes for example methyl, ethyl, n-propyl, *i*-propyl, 2-methyl-ethyl, butyl and its isomers (e.g. n-butyl, *i*-butyl and *t*-butyl); pentyl and its isomers, hexyl and its isomers, heptyl and its isomers, octyl and its isomers, nonyl and its isomers, decyl and its isomers and the like. For example, C₁₋₆alkyl includes all linear, or branched alkyl groups having 1 to 6 carbon atoms, and thus includes for example methyl, ethyl, n-propyl, *i*-propyl, 2-methyl-ethyl, butyl and its isomers (e.g. n-butyl, *i*-butyl and *t*-butyl); pentyl and its isomers, hexyl and its isomers.

When the suffix "ene" is used in conjunction with an alkyl group, i.e. "alkylene", this is intended to mean the alkyl group as defined herein having two single bonds as points of attachment to other groups. As used herein, the term "C₁₋₁₂alkylene", by itself or as part of another substituent, refers to C₁₋₁₂alkyl groups that are divalent, i.e., with two single bonds for attachment to two other groups. Alkylene groups may be linear or branched and may be substituted as indicated herein. Non-limiting examples of alkylene groups include methylene (-CH₂-), ethylene (-CH₂-CH₂-), methylmethylene (-CH(CH₃)-), 1-methyl-ethylene (-CH(CH₃)-CH₂-), n-propylene (-CH₂-CH₂-CH₂-), 2-methylpropylene (-CH₂-CH(CH₃)-CH₂-), 3-methylpropylene (-CH₂-CH₂-CH(CH₃)-), n-butylene (-CH₂-CH₂-CH₂-CH₂-), 2-methylbutylene (-CH₂-CH(CH₃)-CH₂-CH₂-), 4-methylbutylene (-CH₂-CH₂-CH₂-CH(CH₃)-), pentylene and its chain isomers, hexylene and its chain isomers, heptylene and its chain isomers, octylene and its chain isomers, nonylene and its chain isomers, decylene and its chain isomers, undecylene and its chain isomers, dodecylene and its chain isomers.

When the term "alkyl" is used as a suffix following another term, as in "hydroxyalkyl," this is intended to refer to an alkyl group, as defined above, being substituted with one or two (preferably one) substituent(s) selected from the other, specifically-named group, also as defined herein. The term "hydroxyC₁₋₈alkyl" therefore refers to a -R^{a}-OH group wherein R^{a} is C₁₋₈alkylene as defined herein.

The term "C₁₋₆alkoxy" or "C₁₋₆alkyloxy", as a group or part of a group, refers to a group having the Formula -OR^{b} wherein R^{b} is C₁₋₆alkyl as defined herein above. Non-limiting examples of suitable C₁₋₆alkoxy include methoxy, ethoxy, propoxy, isopropoxy, butoxy, isobutoxy, sec-butoxy, tert-butoxy, pentyloxy and hexyloxy.

The term "amino" refers to the group -NH₂.

The terms "C₁₋₆alkylamino" or "mono- or di-C₁₋₆alkylamino", as a group or part of a group, refers to a group of formula -N(R^{k})(Rⁱ) wherein R^{k} and Rⁱ are each independently selected from hydrogen, or C₁₋₆alkyl, wherein at least one of R^{k} or Rⁱ is C₁₋₆alkyl. Thus, alkylamino include mono-alkyl amino group (e.g. mono-C₁₋₆alkylamino group such as methylamino and ethylamino), and di-alkylamino group (e.g. di-C₁₋₆alkylamino group such as dimethylamino and diethylamino). Non-limiting examples of suitable alkylamino groups include n-propylamino, isopropylamino, n-butylamino, *i*-butylamino, sec-butylamino, *t*-butylamino, pentylamino, n-hexylamino, di-n-propylamino, di-*i*-propylamino, ethylmethylamino, methyl-n-propylamino, methyl-i-propylamino, n-butylmethylamino, *i-*butylmethylamino, *t*-butylmethylamino, ethyl-n-propylamino, ethyl-i-propylamino, n-butylethylamino, i-butylethylamino, *t*-butylethylamino, di-n-butylamino, di-*i*-butylamino, methylpentylamino, methylhexylamino, ethylpentylamino, ethylhexylamino, propylpentylamino, propylhexylamino, and the like.

The term "aminoC₁₋₆alkyl", as a group or part of a group, refers to a group of formula -R^{j}-NR^{k}R^{l}wherein R^{j} is C₁₋₆alkylene, R^{k} is hydrogen or C₁₋₆alkyl as defined herein, and R^{l} is hydrogen or C₁₋₆alkyl as defined herein.

The terms "C₁₋₆alkylaminoC₁₋₆alkyl"or "mono- or di-C₁₋₆alkylaminoC₁₋₆alkyl" , as a group or part of a group, refers to a group of formula -R^{j}-N(R^{k})(Rⁱ) wherein R^{k} and Rⁱ are each independently selected from hydrogen, or C₁₋₆alkyl, wherein at least one of R^{k} or Rⁱ is C₁₋₆alkyl and R^{j} is C₁₋₆alkylene.

The term "cycloalkyl", as a group or part of a group, refers to a cyclic alkyl group, that is a monovalent, saturated, hydrocarbyl group having 1 or more cyclic structure, and comprising from 3 to 12 carbon atoms, more preferably from 3 to 9 carbon atoms, more preferably from 3 to 6 carbon atoms, still more preferably from 5 to 6 carbon atoms. Cycloalkyl includes all saturated hydrocarbon groups containing 1 or more rings, including monocyclic or bicyclic groups. The further rings of multi-ring cycloalkyls may be either fused, bridged and/or joined through one or more spiro atoms. When a subscript is used herein following a carbon atom, the subscript refers to the number of carbon atoms that the named group may contain. For example, the term "C₃₋₆cycloalkyl", a cyclic alkyl group comprising from 3 to 6 carbon atoms, more preferably from 5 to 6 carbon atoms. Examples of C₃₋₆cycloalkyl groups include but are not limited to cyclopropyl, cyclobutyl, cyclopentyl, cyclohexyl. Cycloalkyl groups may also be considered to be a subset of homocyclic rings discussed hereinafter. When the suffix "ene" is used in conjunction with a cycloalkyl group, i.e. cycloalkylene, this is intended to mean the cycloalkyl group as defined herein having two single bonds as points of attachment to other groups. Non-limiting examples of "C₃₋₆cycloalkylene" include 1,2-cyclopropylene, 1,1-cyclopropylene, 1,1-cyclobutylene, 1,2-cyclobutylene, 1,3-cyclopentylene, 1,1-cyclopentylene, and 1,4-cyclohexylene.

The term "C₃₋₁₂cycloalkenyl", as a group or part of a group, refers to a non-aromatic mono- or multicyclic hydrocarbyl substituent having the indicated number of carbon atoms and containing at least one carbon-carbon double bond. Non-limiting examples of cycloalkenyl include cyclopentenyl, cyclopentadienyl, cyclohexenyl, cyclohexadienyl, cycloheptenyl, cycloheptadienyl, cycloheptatrienyl, cyclooctenyl, cyclooctadienyl, cyclooctatrienyl, cyclooctatetraenyl, cyclononenyl, cyclononadienyl, cyclodecenyl, cyclodekadienyl and the like.

The term "C₆₋₁₂aryl", as a group or part of a group, refers to a polyunsaturated, aromatic hydrocarbyl group having a single ring (i.e. phenyl) or multiple aromatic rings fused together (e.g. naphthalene), or linked covalently, typically containing 6 to 12 atoms; wherein at least one ring is aromatic. The aromatic ring may optionally include one to two additional rings (either cycloalkyl, heterocyclyl or heteroaryl) fused thereto. Examples of suitable aryl include C₆₋₁₀aryl, more preferably C₆₋₈aryl. Non-limiting examples of C₆₋₁₂aryl comprise phenyl, biphenylyl, biphenylenyl, or 1-or 2-naphthanelyl; 5- or 6-tetralinyl, 1-, 2-, 3-, 4-, 5-, 6-, 7- or 8-azulenyl, 4-, 5-, 6 or 7-indenyl, 4- or 5-indanyl, 5-, 6-, 7- or 8-tetrahydronaphthyl, 1,2,3,4-tetrahydronaphthyl, and 1,4-dihydronaphthyl. When the suffix "ene" is used in conjunction with an aryl group, this is intended to mean the aryl group as defined herein having two single bonds as points of attachment to other groups. Suitable arylene groups include 1,4-phenylene, 1,2-phenylene, 1,3-phenylene, biphenylylene, naphthylene, indenylene, and the like. Where a carbon atom in an aryl group is replaced with a heteroatom, the resultant ring is referred to herein as a heteroaryl ring.

The term "C₆₋₁₀aryleneC₁₋₆alkylene", as a group or part of a group, refers to a group having the Formula -R^{c}-R^{a}- wherein R^{c} is C₆₋₁₀arylene as defined herein and R^{a} is C₁₋₆alkylene as defined herein.

The term "C₆₋₁₂arylC₁₋₁₂alkyl", as a group or part of a group, means a C₁₋₁₂alkyl as defined herein, wherein at least one hydrogen atom is replaced by at least one C₆₋₁₂aryl as defined herein. Non-limiting examples of C₆₋₁₂arylC₁₋₁₂alkyl group include benzyl, phenethyl, dibenzylmethyl, methylphenylmethyl, 3-(2-naphthyl)-butyl, and the like.

The term "aralkyl" as a group or part of a group refers to an aryl-substituted alkyl radical, for example to a C₆₋₁₂arylC₁₋₁₂alkyl as described above. When a subscript is used herein following a carbon atom, the subscript refers to the number of carbon atoms that the named group may contain.

The term "C₁₋₆alkylC₆₋₁₀aryl" as a group or part of a group, refers to a C₆₋₁₀aryl group as defined herein, wherein a hydrogen atom is replaced by a C₁₋₆alkyl as defined herein. The term "alkaryl" as a group or part of a group refers to an alkyl-substituted aryl radical, for example to a C₁₋₆alkylC₆₋₁₀aryl as described above. When a subscript is used herein following a carbon atom, the subscript refers to the number of carbon atoms that the named group may contain.

The term "alkenyl" as a group or part of a group, refers to an unsaturated hydrocarbyl group, which may be linear, or branched, comprising one or more carbon-carbon double bonds. When a subscript is used herein following a carbon atom, the subscript refers to the number of carbon atoms that the named group may contain. For example, the term "C₂₋₂₄alkenyl" refers to an unsaturated hydrocarbyl group, which may be linear, or branched comprising one or more carbon-carbon double bonds and comprising from 2 to 24 carbon atoms. For example, C₂₋₁₀alkenyl includes all linear, or branched alkenyl groups having 2 to 10 carbon atoms. For example, C₂₋₆alkenyl includes all linear, or branched alkenyl groups having 2 to 6 carbon atoms. Examples of alkenyl groups are ethenyl, 2-propenyl, 2-butenyl, 3-butenyl, 2-pentenyl and its isomers, 2-hexenyl and its isomers, 2,4-pentadienyl. and the like. Where alkenyl groups as defined herein are divalent groups having single bonds for attachment to two other groups, they are termed "alkenylene".

The term "alkynyl" by itself or as part of another substituent, refers to an unsaturated hydrocarbyl group, which may be linear, or branched, comprising one or more carbon-carbon triple bonds. When a subscript is used herein following a carbon atom, the subscript refers to the number of carbon atoms that the named group may contain. For example, the term "C₂₋₂₄alkynyl" refers to an unsaturated hydrocarbyl group, which may be linear, or branched comprising one or more carbon-carbon triple bonds and comprising from 2 to 24 carbon atoms. For example, C₂₋₁₀alkynyl includes all linear, or branched alkynyl groups having 2 to 10 carbon atoms. For example, C₂₋₆alkynyl includes all linear, or branched alkynyl groups having 2 to 6 carbon atoms. Non limiting examples of C₂₋₆alkynyl groups include ethynyl, 2-propynyl, 2-butynyl, 3-butynyl, 2-pentynyl and its chain isomers, 2-hexynyl and its chain isomers, and the like. Where alkynyl groups as defined herein are divalent groups having single bonds for attachment to two other groups, they are termed "alkynylene".

As used herein, the term "heteroalkyl" refers to an alkyl group containing one or more S, N, O, P, or Si atoms, meaning an alkyl as defined herein which comprises one or more heteroatoms in the hydrocarbon chain, whereas the heteroatoms may be positioned at the beginning of the hydrocarbon chain, in the hydrocarbon chain or at the end of the hydrocarbon chain. Examples of heteroalkyl include alkoxy, alkoxyalkyl, alkylamino, alkylaminoalkyl, alkylthio, alkylthioalkyl, such as methoxy, methylthio, ethoxy, propoxy, CH₃-O-CH₂-, CH₃-S-CH₂-, CH₃-CH₂-O-CH₂-, CH₃NH-, (CH₃)₂N-, (CH₃)₂-CH₂-NHCH₂-CH₂-, among many other examples.

The term "halo" or "halogen" as a group or part of a group is generic for fluoro, chloro, bromo, and iodo, preferably to F, Cl, Br, and I, more preferably to F, Cl, and Br.

The term "haloC₁₋₆alkyl" as a group or part of a group, refers to a C₁₋₆alkyl group having the meaning as defined above wherein one or more hydrogens are replaced with one or more halogen as defined above. Non-limiting examples of such haloalkyl groups include chloromethyl, 1-bromoethyl, fluoromethyl, difluoromethyl, trifluoromethyl, 1,1,1-trifluoroethyl and the like.

The term "haloC₁₋₆alkoxy", as a group or part of a group, refers to a group of Formula -O-R^{v} wherein R^{v} is haloC₁₋₆alkyl as defined herein. Non-limiting examples of suitable haloC₁₋₆alkoxy include fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy, 2,2,2-trichloroethoxy, trichloromethoxy, 2-bromoethoxy, pentafluoroethyl, 3,3,3-trichloropropoxy, 4,4,4-trichlorobutoxy.

The term "ether" includes both mono and polyethers and refers to groups having a chain containing carbon and oxygen and each of these units preferably comprises 1 to 12 carbons for each oxygen atom. Examples are dimethyl, diethyl and dipropyl ethers, polyethyleneoxide, polyprolyleneoxide, polyethelene glycol, polybuteleneoxide.

The term "C₂₋₂₄alkyl ether" as a group or part of a group, encompasses C₁₋₁₂alkoxyC₁₋₁₂alkyl, and refers to a group having the Formula -R^{a1}-O-R^{b1} wherein R^{b1} is C₁₋₁₂alkyl as defined herein, and R^{a1} is C₁₋₁₂alkylene. The term "C₂₋₁₂alkyl ether" refers to a group having the Formula -R^{a2}-O-R^{b} wherein R^{b} is C₁₋₆alkyl as defined herein, and R^{a1} is C₁₋₆alkylene.

The term "C₃₋₂₄alkyl ether" as a group or part of a group, refers to a group having the Formula -R^{a3}-O-R^{b3} wherein R^{b3} is C₂₋₁₂alkenyl as defined herein, and R^{a3} is C₁₋₁₂alkylene; or wherein R^{b3} is C₁₋₁₂alkyl as defined herein, and R^{a3} is C₂₋₁₂alkenylene, or wherein R^{b3} is C₂₋₁₂alkenyl as defined herein, and R^{a3} is C₂₋₁₂alkenylene. The term "C₃₋₁₂alkenyl ether" as a group or part of a group refers to a group having the Formula -R^{a4}-O-R^{b4} wherein R^{b4} is C₂₋₆alkenyl as defined herein, and R^{a4} is C₁₋₆alkylene; or wherein R^{b4} is C₁₋₆alkyl as defined herein, and R^{a4} is C₂₋₆alkenylene, or wherein R^{b4} is C₂₋₆alkenyl as defined herein, and R^{a4} is C₂₋₆alkenylene.

The term "C₁₋₆alkylthio", as a group or part of a group, refers to a group having the Formula -S-R^{b} wherein R^{b} is C₁₋₆alkyl as defined herein above. Non-limiting examples of C₁₋₆alkylthio groups include methylthio (-SCH₃), ethylthio (-SCH₂CH₃), n-propylthio, isopropylthio, n-butylthio, isobutylthio, sec-butylthio, tert-butylthio, and the like.

The terms "heterocycloakyl" or "heterocyclyl" or "heterocyclo", as a group or part of a group, refer to non-aromatic, fully saturated or partially unsaturated cyclic groups (for example, 3 to 7 member monocyclic, 7 to 11 member bicyclic, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from N, O and/or S, where the N and S heteroatoms may optionally be oxidized and the N heteroatoms may optionally be quaternized. The heterocyclic group may be attached at any heteroatom or carbon atom of the ring or ring system, where valence allows. The rings of multi-ring heterocycles may be fused, bridged and/or joined through one or more spiro atoms.

Non limiting exemplary heterocyclic groups include aziridinyl, oxiranyl, thiiranyl, piperidinyl, azetidinyl, 2-imidazolinyl, pyrazolidinyl imidazolidinyl, isoxazolinyl, oxazolidinyl, isoxazolidinyl, thiazolidinyl, isothiazolidinyl, piperidinyl, succinimidyl, 3H-indolyl, indolinyl, isoindolinyl, 2H-pyrrolyl, 1-pyrrolinyl, 2-pyrrolinyl, 3-pyrrolinyl, pyrrolidinyl, 4H-quinolizinyl, 2-oxopiperazinyl, piperazinyl, homopiperazinyl, 2-pyrazolinyl, 3-pyrazolinyl, tetrahydro-2H-pyranyl, 2H-pyranyl, 4H-pyranyl, 3,4-dihydro-2H-pyranyl, oxetanyl, thietanyl, 3-dioxolanyl, 1,4-dioxanyl, 2,5-dioximidazolidinyl, 2-oxopiperidinyl, 2-oxopyrrolodinyl, indolinyl, tetrahydropyranyl, tetrahydrofuranyl, tetrahydrothiophenyl, tetrahydroquinolinyl, tetrahydroisoquinolin-1-yl, tetrahydroisoquinolin-2-yl, tetrahydroisoquinolin-3-yl, tetrahydroisoquinolin-4-yl, thiomorpholin-4-yl, thiomorpholin-4-ylsulfoxide, thiomorpholin-4-ylsulfone, 1,3-dioxolanyl, 1,4-oxathianyl, 1,4-dithianyl, 1,3,5-trioxanyl, 1H-pyrrolizinyl, tetrahydro-1,1-dioxothiophenyl, N- formylpiperazinyl, and morpholin-4-yl.

The term "heteroaryl" as a group or part of a group, refers but is not limited to 5 to 12 carbon-atom aromatic rings or ring systems containing 1 to 2 rings which are fused together or linked covalently, typically containing 5 to 6 atoms; at least one of which is aromatic in which one or more carbon atoms in one or more of these rings can be replaced by N, O and/or S atoms where the N and S heteroatoms may optionally be oxidized and the N heteroatoms may optionally be quaternized. Such rings may be fused to an aryl, cycloalkyl, heteroaryl or heterocyclyl ring. Non-limiting examples of such heteroaryl, include: pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, thienopyridinyl, purinyl, imidazo[1,2-a]pyridinyl, 6-oxo-pyridazin-1(6H)-yl, 2-oxopyridin-1(2H)-yl, 6-oxo-pyridazin-1(6H)-yl, 2-oxopyridin-1(2H)-yl, 1,3-benzodioxolyl, quinolinyl, isoquinolinyl, cinnolinyl, quinazolinyl, quinoxalinyl; preferably said heteroaryl group is selected from the group consisting of pyridyl, pyrazinyl, pyrazolyl, pyrrolyl, imidazolyl, benzimidazolyl, pyrimidinyl, triazolyl and thiazolyl.

The term "pyrrolyl" (also called azolyl) as used herein includes pyrrol-1-yl, pyrrol-2-yl and pyrrol-3-yl. The term "furanyl" (also called "furyl") as used herein includes furan-2-yl and furan-3-yl (also called furan-2-yl and furan-3-yl). The term "thiophenyl" (also called "thienyl") as used herein includes thiophen-2-yl and thiophen-3-yl (also called thien-2-yl and thien-3-yl). The term "pyrazolyl" (also called 1H-pyrazolyl and 1,2-diazolyl) as used herein includes pyrazol-1-yl, pyrazol-3-yl, pyrazol-4-yl and pyrazol-5-yl. The term "imidazolyl" as used herein includes imidazol-1-yl, imidazol-2-yl, imidazol-4-yl and imidazol-5-yl. The term "oxazolyl" (also called 1,3-oxazolyl) as used herein includes oxazol-2-yl; oxazol-4-yl and oxazol-5-yl. The term "isoxazolyl" (also called 1,2-oxazolyl), as used herein includes isoxazol-3-yl, isoxazol-4-yl, and isoxazol-5-yl. The term "thiazolyl" (also called 1,3-thiazolyl),as used herein includes thiazol-2-yl, thiazol-4-yl and thiazol-5-yl (also called 2-thiazolyl, 4-thiazolyl and 5-thiazolyl). The term "isothiazolyl" (also called 1,2-thiazolyl) as used herein includes isothiazol-3-yl, isothiazol-4-yl, and isothiazol-5-yl. The term "triazolyl" as used herein includes 1H-triazolyl and 4H-1,2,4-triazolyl, "1H-triazolyl" includes 1H-1,2,3-triazol-1-yl, 1H-1,2,3-triazol-4-yl, 1H-1,2,3-triazol-5-yl, 1H-1,2,4-triazol-1-yl, 1H-1,2,4-triazol-3-yl and 1H-1,2,4-triazol-5-yl. "4H-1,2,4-triazolyl" includes 4H-1,2,4-triazol-4-yl, and 4H-1,2,4-triazol-3-yl. The term "oxadiazolyl" as used herein includes 1,2,3-oxadiazol-4-yl, 1,2,3-oxadiazol-5-yl, 1,2,4-oxadiazol-3-yl, 1,2,4-oxadiazol-5-yl, 1,2,5-oxadiazol-3-yl and 1,3,4-oxadiazol-2-yl. The term "thiadiazolyl" as used herein includes 1,2,3-thiadiazol-4-yl, 1,2,3-thiadiazol-5-yl, 1,2,4-thiadiazol-3-yl, 1,2,4-thiadiazol-5-yl, 1,2,5-thiadiazol-3-yl (also called furazan-3-yl) and 1,3,4-thiadiazol-2-yl. The term "tetrazolyl" as used herein includes 1H-tetrazol-1-yl, 1H-tetrazol-5-yl, 2H-tetrazol-2-yl, and 2H-tetrazol-5-yl. The term "oxatriazolyl" as used herein includes 1,2,3,4-oxatriazol-5-yl and 1,2,3,5-oxatriazol-4-yl. The term "thiatriazolyl" as used herein includes 1,2,3,4-thiatriazol-5-yl and 1,2,3,5-thiatriazol-4-yl. The term "pyridinyl" (also called "pyridyl") as used herein includes pyridin-2-yl, pyridin-3-yl and pyridin-4-yl (also called 2-pyridyl, 3-pyridyl and 4-pyridyl). The term "pyrimidyl" as used herein includes pyrimid-2-yl, pyrimid-4-yl, pyrimid-5-yl and pyrimid-6-yl. The term "pyrazinyl" as used herein includes pyrazin-2-yl and pyrazin-3-yl. The term "pyridazinyl as used herein includes pyridazin-3-yl and pyridazin-4-yl. The term "oxazinyl" (also called "1,4-oxazinyl") as used herein includes 1,4-oxazin-4-yl and 1,4-oxazin-5-yl. The term "dioxinyl" (also called "1,4-dioxinyl") as used herein includes 1,4-dioxin-2-yl and 1,4-dioxin-3-yl. The term "thiazinyl" (also called "1,4-thiazinyl") as used herein includes 1,4-thiazin-2-yl, 1,4-thiazin-3-yl, 1,4-thiazin-4-yl, 1,4-thiazin-5-yl and 1,4-thiazin-6-yl. The term "triazinyl" as used herein includes 1,3,5-triazin-2-yl, 1,2,4-triazin-3-yl, 1,2,4-triazin-5-yl, 1,2,4-triazin-6-yl, 1,2,3-triazin-4-yl and 1,2,3-triazin-5-yl. The term "imidazo[2,1-b][1,3]thiazolyl" as used herein includes imidazo[2,1-b][1,3]thiazoi-2-yl, imidazo[2,1-b][1,3]thiazol-3-yl, imidazo[2,1-b][1,3]thiazol-5-yl and imidazo[2,1-b][1,3]thiazol-6-yl. The term "thieno[3,2-b]furanyl" as used herein includes thieno[3,2-b]furan-2-yl, thieno[3,2-b]furan-3-yl, thieno[3,2-b]furan-4-yl, and thieno[3,2-b]furan-5-yl. The term "thieno[3,2-b]thiophenyl" as used herein includes thieno[3,2-b]thien-2-yl, thieno[3,2-b]thien-3-yl, thieno[3,2-b]thien-5-yl and thieno[3,2-b]thien-6-yl. The term "thieno[2,3-d][1,3]thiazolyl" as used herein includes thieno[2,3-d][1,3]thiazol-2-yl, thieno[2,3-d][1,3]thiazol-5-yl and thieno[2,3-d][1,3]thiazol-6-yl. The term "thieno[2,3-d]imidazolyl" as used herein includes thieno[2,3-d]imidazol-2-yl, thieno[2,3-d]imidazol-4-yl and thieno[2,3-d]imidazol-5-yl. The term "tetrazolo[1,5-a]pyridinyl" as used herein includes tetrazolo[1,5-a]pyridine-5-yl, tetrazolo[1,5-a]pyridine-6-yl, tetrazolo[1,5-a]pyridine-7-yl, and tetrazolo[1,5-a]pyridine-8-yl. The term "indolyl" as used herein includes indol-1-yl, indol-2-yl, indol-3-yl,-indol-4-yl, indol-5-yl, indol-6-yl and indol-7-yl. The term "indolizinyl" as used herein includes indolizin-1-yl, indolizin-2-yl, indolizin-3-yl, indolizin-5-yl, indolizin-6-yl, indolizin-7-yl, and indolizin-8-yl. The term "isoindolyl" as used herein includes isoindol-1-yl, isoindol-2-yl, isoindol-3-yl, isoindol-4-yl, isoindol-5-yl, isoindol-6-yl and isoindol-7-yl. The term "benzofuranyl" (also called benzo[b]furanyl) as used herein includes benzofuran-2-yl, benzofuran-3-yl, benzofuran-4-yl, benzofuran-5-yl, benzofuran-6-yl and benzofuran-7-yl. The term "isobenzofuranyl" (also called benzo[c]furanyl) as used herein includes isobenzofuran-1-yl, isobenzofuran-3-yl, isobenzofuran-4-yl, isobenzofuran-5-yl, isobenzofuran-6-yl and isobenzofuran-7-yl. The term "benzothiophenyl" (also called benzo[b]thienyl) as used herein includes 2-benzo[b]thiophenyl, 3-benzo[b]thiophenyl, 4-benzo[b]thiophenyl, 5-benzo[b]thiophenyl, 6-benzo[b]thiophenyl and -7-benzo[b]thiophenyl (also called benzothien-2-yl, benzothien-3-yl, benzothien-4-yl, benzothien-5-yl, benzothien-6-yl and benzothien-7-yl). The term "isobenzothiophenyl" (also called benzo[c]thienyl) as used herein includes isobenzothien-1-yl, isobenzothien-3-yl, isobenzothien-4-yl, isobenzothien-5-yl, isobenzothien-6-yl and isobenzothien-7-yl. The term "indazolyl" (also called 1H-indazolyl or 2-azaindolyl) as used herein includes 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 2H-indazol-3-yl, 2H-indazol-4-yl, 2H-indazol-5-yl, 2H-indazol-6-yl, and 2H-indazol-7-yl. The term "benzimidazolyl" as used herein includes benzimidazol-1-yl, benzimidazol-2-yl, benzimidazol-4-yl, benzimidazol-5-yl, benzimidazol-6-yl and benzimidazol-7-yl. The term "1,3-benzoxazolyl" as used herein includes 1,3-benzoxazol-2-yl, 1,3-benzoxazol-4-yl, 1,3-benzoxazol-5-yl, 1,3-benzoxazol-6-yl and 1,3-benzoxazol-7-yl. The term "1,2-benzisoxazolyl" as used herein includes 1,2-benzisoxazol-3-yl, 1,2-benzisoxazol-4-yl, 1,2-benzisoxazol-5-yl, 1,2-benzisoxazol-6-yl and 1,2-benzisoxazol-7-yl. The term "2,1-benzisoxazolyl" as used herein includes 2,1-benzisoxazol-3-yl, 2,1-benzisoxazol-4-yl, 2,1-benzisoxazol-5-yl, 2,1-benzisoxazol-6-yl and 2,1-benzisoxazol-7-yl. The term "1,3-benzothiazolyl" as used herein includes 1,3-benzothiazol-2-yl, 1,3-benzothiazol-4-yl, 1,3-benzothiazol-5-yl, 1,3-benzothiazol-6-yl and 1,3-benzothiazol-7-yl. The term "1,2-benzoisothiazolyl" as used herein includes 1,2-benzisothiazol-3-yl, 1,2-benzisothiazol-4-yl, 1,2-benzisothiazol-5-yl, 1,2-benzisothiazol-6-yl and 1,2-benzisothiazol-7-yl. The term "2,1-benzoisothiazolyl" as used herein includes 2,1-benzisothiazol-3-yl, 2,1-benzisothiazol-4-yl, 2,1-benzisothiazol-5-yl, 2,1-benzisothiazol-6-yl and 2,1-benzisothiazol-7-yl. The term "benzotriazolyl" as used herein includes benzotriazol-1-yl, benzotriazol-4-yl, benzotriazol-5-yl, benzotriazol-6-yl and benzotriazol-7-yl. The term "1,2,3-benzoxadiazolyl" as used herein includes 1,2,3-benzoxadiazol-4-yl, 1,2,3-benzoxadiazol-5-yl, 1,2,3-benzoxadiazol-6-yl and 1,2,3-benzoxadiazol-7-yl. The term "2,1,3-benzoxadiazolyl" as used herein includes 2,1,3-benzoxadiazol-4-yl, 2,1,3-benzoxadiazol-5-yl, 2,1,3-benzoxadiazol-6-yl and 2,1,3-benzoxadiazol-7-yl. The term "1,2,3-benzothiadiazolyl" as used herein includes 1,2,3-benzothiadiazol-4-yl, 1,2,3-benzothiadiazol-5-yl, 1,2,3-benzothiadiazol-6-yl and 1,2,3-benzothiadiazol-7-yl. The term "2,1,3-benzothiadiazolyl" as used herein includes 2,1,3-benzothiadiazol-4-yl, 2,1,3-benzothiadiazol-5-yl, 2,1,3-benzothiadiazol-6-yl and 2,1,3-benzothiadiazol-7-yl. The term "thienopyridinyl" as used herein includes thieno[2,3-b]pyridinyl, thieno[2,3-c]pyridinyl, thieno[3,2-c]pyridinyl and thieno[3,2-b]pyridinyl. The term "purinyl" as used herein includes purin-2-yl, purin-6-yl, purin-7-yl and purin-8-yl. The term "imidazo[1,2-a]pyridinyl", as used herein includes imidazo[1,2-a]pyridin-2-yl, imidazo[1,2-a]pyridin-3-yl, imidazo[1,2-a]pyridin-4-yl, imidazo[1,2-a]pyridin-5-yl, imidazo[1,2-a]pyridin-6-yl and imidazo[1,2-a]pyridin-7-yl. The term "1,3-benzodioxolyl", as used herein includes 1,3-benzodioxol-4-yl, 1,3-benzodioxol-5-yl, 1,3-benzodioxol-6-yl, and 1,3-benzodioxol-7-yl. The term "quinolinyl" as used herein includes quinolin-2-yl, quinolin-3-yl, quinolin-4-yl, quinolin-5-yl, quinolin-6-yl, quinolin-7-yl and quinolin-8-yl. The term "isoquinolinyl" as used herein includes isoquinolin-1-yl, isoquinolin-3-yl, isoquinolin-4-yl, isoquinolin-5-yl, isoquinolin-6-yl, isoquinolin-7-yl and isoquinolin-8-yl. The term "cinnolinyl" as used herein includes cinnolin-3-yl, cinnolin-4-yl, cinnolin-5-yl, cinnolin-6-yl, cinnolin-7-yl and cinnolin-8-yl. The term "quinazolinyl" as used herein includes quinazolin-2-yl, quinazolin-4-yl, quinazolin-5-yl, quinazolin-6-yl, quinazolin-7-yl and quinazolin-8-yl. The term "quinoxalinyl". as used herein includes quinoxalin-2-yl, quinoxalin-5-yl, and quinoxalin-6-yl.

By a "functional group" is meant a group that contains one or more reactive moieties. Examples of functional groups include halo, hydroxyl, sulfhydryl, C₁₋₂₄alkoxy, C₂₋₂₄ alkenyloxy, C₂₋₂₄alkynyloxy, C₆₋₂₀aryloxy, acyl (including C₁₋₂₄alkylcarbonyl (-C(=O)-alkyl) and C₆₋₂₀arylcarbonyl (-C(=O)-aryl)), acyloxy (-O-acyl), C₁₋₂₄alkoxycarbonyl (-C(=O)-O-alkyl), C₆₋₂₀aryloxycarbonyl (-C(=O)-O-aryl), halocarbonyl (-C(=O)-X^{a} where X^{a} is halo), C₁₋₂₄alkylcarbonato (-O-C(=O)-O-C₁₋₂₄alkyl), C₆₋₂₀arylcarbonato (-O-C(=O)-O-aryl), carboxy (-C(=O)OH), carboxylato (-C(=O)-O⁻), carbamoyl (-C(=O)-NH₂), mono-substituted C₁₋₂₄alkylcarbamoyl (-(C=O)-NH(C₁₋₂₄alkyl)), di-substituted C₁₋₂₄alkylcarbamoyl (-C(=O)-N(C₁₋₂₄alkyl)₂), mono-substituted arylcarbamoyl (-C(=O)-NH-aryl), thiocarbamoyl (-C(=S)-NH₂), carbamido (-NH-C(=O)-NH₂), cyano (-C=N), isocyano (-N+=C-), cyanato (-O-C=N), isocyanato (-N=C=O), isothiocyanato (-S-C=N), azido (-N=N+=N-), formyl (-C(=O)-H), thioformyl (-C(=S)-H), amino (-NH₂), mono- and di-(C₁₋₂₄alkyl)-substituted amino, mono- and di-(C₆₋₂₀aryl)-substituted amino, C₁₋₂₄alkylamido (-NH-C(=O)-alkyl), C₆₋₂₀arylamido (-NH-C(=O)-aryl), imino (-CR^{x}=NH wherein R^{x} is hydrogen, C₁₋₂₄alkyl, C₆₋₂₀aryl, C₆₋₂₀alkaryl, C₆₋₂₀aralkyl, etc.), C₁₋₂₄alkylimino (-CR^{y}=N(C₁₋₂₄alkyl), wherein R^{y} is hydrogen, C₁₋₂₄alkyl, C₆₋₂₀aryl, C₆₋₂₀alkaryl, etc.), C₆₋₂₀arylimino (-CR^{v}=N(C₆₋₂₀aryl), wherein R^{v} is hydrogen, C₁₋₂₄alkyl, C₆₋₂₀aryl, C₆₋₂₀alkaryl, etc.), nitro (-NO₂), nitroso (-NO), sulfo (-SO₂-OH), sulfonato (-SO₂-O-), C₁₋₂₄alkylsulfanyl (-S-C₁₋₂₄alkyl; also termed "C₁₋₂₄alkylthio"), arylsulfanyl (-S-aryl; also termed "arylthio"), C₁₋₂₄alkylsulfinyl (-(SO)-C₁₋₂₄alkyl), C₆₋₂₀arylsulfinyl (-(SO)-C₆₋₂₀aryl), C₁₋₂₄alkylsulfonyl (-SO₂-C₁₋₂₄alkyl), C₆₋₂₀arylsulfonyl (-SO₂-C₆₋₂₀aryl), phosphono (-P(O)(OH)₂), phosphonato (-P(O)(O-)₂), phosphinato (-P(O)(O-)), phospho (-PO₂), and phosphino (-PH₂), mono- and di-(C₁₋₂₄alkyl)-substituted phosphino, and mono- and di-(C₆₋₂₀aryl)-substituted phosphino.

The terms described above and others used in the specification are well understood to those in the art.

In a first aspect, the present invention provides a drier composition for an autoxidizable alkyd based coating composition, comprising:
a) at least one Fe complex comprising Fe and at least one nitrogen donor ligand, wherein said at least nitrogen donor ligand is selected from the group comprising tridentate, tetradentate, pentadentate and hexadentate nitrogen donor ligands;
b) at least one metal salt of a carboxylic acid, wherein the metal is selected from the group comprising: Mn, Ce, V, and Cu; and
c) at least one ligand comprising at least one moiety selected from the group comprising 1,4,7-tri-azacyclononanyl, 2,2'-bipyridyl, 1,10-phenantrolinyl, imidazolyl, pyrazolyl, porphyrinyl, aliphatic, cycloaliphatic, and aromatic amines.

As used herein, the term "drier" (which is also referred to synonymously as "siccative" when in solution) refers to organometallic compounds that are soluble in organic solvents and binders. They are added to unsaturated oils and binders in order to appreciably reduce their drying times, i.e. the transition of their liquid films to the solid phase. Driers are available either as solids or in solution (siccatives). Suitable solvents are organic solvents, fatty acid esters and binders. The driers are present in amounts expressed as weight percent of the metal based on the weight of binder solids (or resin) unless stated otherwise.

As used herein, the term "drier composition" refers to the mixture of driers as presently claimed. The drier composition according to the invention comprises several drier compounds. The inventors have found that the present selection of driers in an alkyd-based coating composition improves the drying speed of the coating composition while simultaneously reducing skin formation.

According to an embodiment of the invention, the present drier composition comprises at least one Fe complex comprising Fe and at least one nitrogen donor ligand, wherein said at least nitrogen donor ligand is selected from the group comprising tridentate, tetradentate, pentadentate and hexadentate nitrogen donor ligands; preferably selected from the group comprising tetradentate, pentadentate, and hexadentate nitrogen donor ligands.

Preferably, the ligand in a) is a ligand of formula (I) which belong to the bispidon class; wherein 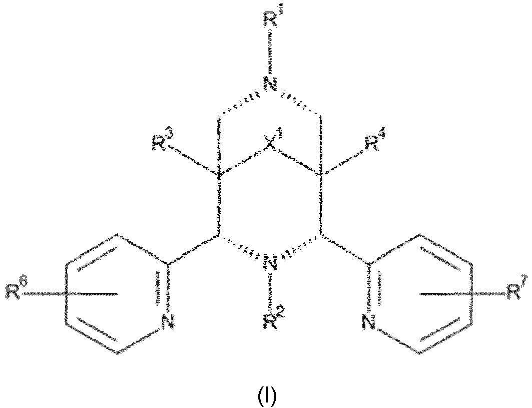
R¹ is selected from the group consisting of C₁₋₂₄alkyl, C₆₋₁₀aryl, a group containing one or two heteroatoms (e.g. N, O, or S) capable of coordinating to the Fe metal; wherein R¹ is optionally substituted with one or more substituents, each independently selected from the group consisting of C₁₋₁₂alkyl, C₆₋₁₀aryl, C₆₋₁₀arylC₁₋₆alkyl; preferably said group containing one or two heteroatoms (e.g. N, O, or S) capable of coordinating to the Fe metal, is selected from heteroaryl, heteroarylC₁₋₆alkyl, C₁₋₆alkylaminoC₁₋₆alkyl such as -CH₂-CH₂-N(CH₃)₂; wherein heteroaryl is selected from the group consisting of pyridyl, pyrazinyl, pyrazolyl, pyrrolyl, imidazolyl, benzimidazolyl, pyrimidinyl, triazolyl and thiazolyl, preferably pyridin-2yl;
preferably R¹ is selected from the group consisting of C₁₋₂₄alkyl, C₆₋₁₀aryl, heteroaryl, C₁₋₆alkylaminoC₁₋₆alkyl, and heteroarylC₁₋₆alkyl, wherein heteroaryl is selected from the group consisting of pyridyl, pyrazinyl, pyrazolyl, pyrrolyl, imidazolyl, benzimidazolyl, pyrimidinyl, triazolyl and thiazolyl, preferably pyridin-2yl; wherein R¹ is optionally substituted with one or more substituents, each independently selected from the group consisting of C₁₋₁₂alkyl, C₆₋₁₀aryl, C₆₋₁₀arylC₁₋₆alkyl;
R² is selected from the group consisting of C₁₋₂₄alkyl, C₆₋₁₀aryl, heteroaryl, C₁₋₆alkylaminoC₁₋₆alkyl, and heteroarylC₁₋₆alkyl, wherein heteroaryl is selected from the group consisting of pyridyl, pyrazinyl, pyrazolyl, pyrrolyl, imidazolyl, benzimidazolyl, pyrimidinyl, triazolyl and thiazolyl, preferably pyridin-2yl; wherein R² is optionally substituted with one or more substituents, each independently selected from the group consisting of C₁₋₁₂alkyl, C₆₋₁₀aryl, C₆₋₁₀arylC₁₋₆alkyl;
R³ is selected from the group consisting of-H, C₁₋₁₂alkyl, C₁₋₁₂alkyl-O-C₁₋₁₂alkyl, C₁₋₁₂alkyl-O-C₆₋₁₀aryl, C₆₋₁₀aryl, hydroxyC₁₋₁₂alkyl, and -(CH₂)ₘC(O)OR⁵; wherein R⁵ is selected from -H, C₁₋₄alkyl, C₆₋₁₀aryl, or C₁₋₄alkylC₆₋₁₀aryl, and wherein m is an integer selected from 0, 1, 2, 3, or 4; wherein R³ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy;
R⁴ is selected from the group consisting of-H, C₁₋₁₂alkyl, C₁₋₁₂alkyl-O-C₁₋₁₂alkyl, C₁₋₁₂alkyl-O-C₆₋₁₀aryl, C₆₋₁₀aryl, hydroxyC₁₋₁₂alkyl, and -(CH₂)ₘC(O)OR⁵⁰; wherein R⁵⁰ is selected from -H, C₁₋₄alkyl, C₆₋₁₀aryl, or C₁₋₄alkylC₆₋₁₀aryl, and wherein m is an integer selected from 0, 1, 2, 3, or 4; wherein R⁴ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy;
R⁶ is selected from the group consisting of -H, halogen, -OH, C₁₋₆alkoxy, -NH-C(O)-H, -NH-C(O)-C₁₋₁₂alkyl, -NH₂, -NH-C₁₋₁₂alkyl, and C₁₋₁₂alkyl; wherein R⁶ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy;
R⁷ is selected from the group consisting of -H, halogen, -OH, C₁₋₆alkoxy, -NH-C(O)-H, -NH-C(O)-C₁₋₁₂alkyl, -NH₂, -NH-C₁₋₁₂alkyl, and C₁₋₁₂alkyl; wherein R⁷ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy;
X¹ is selected from -C(O)- or -[C(R⁸)₂]ₙ-; wherein n is an integer selected from 0, 1, 2 or 3, and each R⁸ is independently selected from the group consisting of-OH, -H, C₁₋₁₂alkoxy and C₁₋₁₂alkyl; wherein R⁸ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy.

In some embodiments, R¹ is selected from the group consisting of C₁₋₂₀alkyl, C₆₋₁₀aryl, heteroaryl, C₁₋₄alkylaminoC₁₋₄alkyl, heteroarylC₁₋₆alkyl, wherein heteroaryl is selected from the group consisting of pyridyl, and pyrazinyl, preferably pyridin-2yl; wherein R¹ is optionally substituted with one or more substituents, each independently selected from the group consisting of C₁₋₆alkyl, C₆₋₁₀aryl, C₆₋₁₀arylC₁₋₄alkyl;
R² is selected from the group consisting of C₁₋₂₀alkyl, C₆₋₁₀aryl, heteroaryl, C₁₋₄alkylaminoC₁₋₄alkyl, heteroarylC₁₋₆alkyl, wherein heteroaryl is selected from the group consisting of pyridyl, and pyrazinyl, preferably pyridin-2yl; wherein R² is optionally substituted with one or more substituents, each independently selected from the group consisting of C₁₋₆alkyl, C₆₋₁₀aryl, C₆₋₁₀arylC₁₋₄alkyl;
R³ is selected from the group consisting of-H, C₁₋₆alkyl, C₁₋₆alkyl-O-C₁₋₆alkyl, C₁₋₆alkyl-O-C₆₋₁₀aryl, C₆₋₁₀aryl, hydroxyC₁₋₆alkyl, and -(CH₂)ₘC(O)OR⁵; wherein R⁵ is selected from -H, C₁₋₄alkyl, C₆₋₁₀aryl, or C₁₋₄alkylC₆₋₁₀aryl, and wherein m is an integer selected from 0, 1, 2, 3, or 4;
R⁴ is selected from the group consisting of-H, C₁₋₆alkyl, C₁₋₆alkyl-O-C₁₋₆alkyl, C₁₋₆alkyl-O-C₆₋₁₀aryl, C₆₋₁₀aryl, hydroxyC₁₋₆alkyl, and -(CH₂)ₘC(O)OR⁵⁰; wherein R⁵⁰ is selected from-H, C₁₋₄alkyl, C₆₋₁₀aryl, or C₁₋₄alkylC₆₋₁₀aryl, and wherein m is an integer selected from 0, 1, 2, 3, or 4;
R⁶ is selected from the group consisting of -H, halogen, -OH, C₁₋₄alkoxy, -NH-C(O)-H, -NH-C(O)-C₁₋₄alkyl, -NH₂, -NH-C₁₋₄alkyl, and C₁₋₄alkyl;
R⁷ is selected from the group consisting of-H, halogen, -OH, C₁₋₄alkoxy, -NH-C(O)-H, -NH-C(O)-C₁₋₄alkyl, -NH₂, -NH-C₁₋₄alkyl, and C₁₋₄alkyl;
X¹ is selected from -C(O)- or -[C(R⁸)₂]ₙ-; wherein n is an integer selected from 0, 1, 2 or 3, and each R⁸ is independently selected from the group consisting of -H, halogen, -OH, C₁₋₆alkoxy and C₁₋₆alkyl.

In some embodiments, R¹ is selected from the group consisting of C₁₋₂₀alkyl, C₆₋₁₀aryl, pyridyl, C₁₋₂alkylaminoC₁₋₂alkyl, wherein R¹ is optionally substituted with one or more substituents, each independently selected from the group consisting of C₁₋₄alkyl, phenyl, benzyl;
R² is selected from the group consisting of C₁₋₂₀alkyl, C₆₋₁₀aryl, pyridyl, C₁₋₂alkylaminoC₁₋₂alkyl, wherein R² is optionally substituted with one or more substituents, each independently selected from the group consisting of C₁₋₄alkyl, phenyl, benzyl;
R³ is selected from the group consisting of C₁₋₆alkyl-O-C₁₋₆alkyl, C₁₋₆alkyl-O-C₆₋₁₀aryl, C₆₋₁₀aryl, hydroxyC₁₋₆alkyl, and -(CH₂)ₘC(O)OR⁵; wherein R⁵ is selected from -H, C₁₋₄alkyl, C₆₋₁₀aryl, or C₁₋₄alkylC₆₋₁₀aryl, and wherein m is an integer selected from 0, 1, 2, or 3;
R⁴ is selected from the group consisting of C₁₋₆alkyl-O-C₁₋₆alkyl, C₁₋₆alkyl-O-C₆₋₁₀aryl, C₆₋₁₀aryl, hydroxyC₁₋₆alkyl, and -(CH₂)ₘC(O)OR⁵⁰; wherein R⁵⁰ is selected from -H, C₁₋₄alkyl, C₆₋₁₀aryl, or C₁₋₄alkylC₆₋₁₀aryl, and wherein m is an integer selected from 0, 1, 2, or 3;
R⁶ is selected from the group consisting of -H, halogen, -OH, C₁₋₄alkoxy, -NH-C(O)-H, -NH-C(O)-C₁₋₄alkyl, -NH₂, -NH-C₁₋₄alkyl, and C₁₋₄alkyl;
R⁷ is selected from the group consisting of -H, halogen, -OH, C₁₋₄alkoxy, -NH-C(O)-H, -NH-C(O)-C₁₋₄alkyl, -NH₂, -NH-C₁₋₄alkyl, and C₁₋₄alkyl;
X¹ is selected from -C(O)- or -[C(R⁸)₂]ₙ-; wherein n is an integer selected from 0, 1, 2 or 3, and each R⁸ is independently selected from the group consisting of-H, -OH, C₁₋₆alkoxy and C₁₋₆alkyl

Preferably, the nitrogen donor ligand is a compound of formula (I);
wherein R¹ and R² are each independently selected from the group consisting of C₁₋₂₄alkyl, C₆₋₁₀aryl, heteroaryl, C₁₋₂alkylaminoC₁₋₂alkyl, and heteroarylC₁₋₆alkyl; wherein heteroaryl is selected from the group consisting of pyridyl, pyrazinyl, and pyrazolyl ;
R³ and R⁴ are each independently selected from the group consisting of-H, C₁₋₈alkyl, C₁₋₈alkyl-O-C₁₋salkyl, C₁₋₈alkyl-O-C₆₋₁₀aryl, C₆₋₁₀aryl, hydroxyC₁₋₈alkyl, and -(CH₂)ₘC(O)OR⁵; R⁵ is selected from -H, C₁₋₄alkyl, C₆₋₁₀aryl, or C₁₋₄alkylC₆₋₁₀aryl, and m is an integer selected from 0, 1, 2, 3, or 4;
each R⁶ and R⁷ are independently selected from the group consisting of -H, -F, -CI, -Br, -OH, C₁₋₄alkoxy, -NH-C(O)-H, -NH-C(O)-C₁₋₄alkyl, -NH₂, -NH-C₁₋₄alkyl, and C₁₋₄alkyl; and,
X¹ is selected from -C(O)- or -[C(R⁸)₂]ₙ-; wherein n is an integer selected from 0, 1, 2 or 3, and each R⁸ is independently selected from the group consisting of-H, -OH, C₁₋₄alkoxy and C₁₋₄alkyl.
wherein R¹ and R² are each independently selected from the group comprising C₁₋₂₄alkyl, C₆₋₁₂aryl, heteroaryl, heteroarylC₁₋₆alkyl, and -CH₂-CH₂-N(CH₃)₂, wherein heteroaryl is selected from the group comprising pyridyl, pyrazinyl, pyrazolyl, pyrrolyl, imidazolyl, benzimidazolyl, pyrimidinyl, triazolyl and thiazolyl;
R³ and R⁴ are each independently selected from the group comprising -H, C₁₋₈alkyl, C₁₋₈alkyl-O-C₁₋₈alkylene, C₁₋₈alkyl-O-C₆₋₁₂arylene, C₆₋₁₂aryl-O-C₁₋₈alkylene, C₆₋₁₂aryl, C₁₋₈hydroxyalkyl, and -(CH₂)ₘC(O)OR⁵, wherein R⁵ is selected from -H or C₁₋₄alkyl, m is an integer selected from 0 to 4;
each R⁶ and R⁷ are independently selected from the group comprising -H, -F, -CI, -Br, -OH, C₁₋₄alkoxy, -NH-C(O)-H, -NH-C(O)-C₁₋₄alkyl, -NH₂, -NH-C₁₋₄alkyl, and C₁₋₄alkyl;
X¹ is selected from -C(O)- or -[C(R⁸)₂]ₙ- wherein n is an integer selected from 0 to 3, and each R⁸ is independently selected from the group comprising -H, -OH, C₁₋₄alkoxy and C₁₋₄alkyl;
Preferably R³ and R⁴ are each independently selected from the group comprising -C(O)-O-CH₃, -C(O)-O-CH₂-CH₃, -C(O)-O-CH₂-C₆H₅ and CH₂OH.

Preferably X¹ is C=O.

Preferred groups for R¹ and R² are selected from the group comprising CH₃, -C₂H₅, -C₃H₇, benzyl, - C₄H₉, -C₆H₁₃, -C₈H₁₇, -C₁₂H₂₅, and -C₁₈H_{37;} -CH₂-pyridyl, and pyridin-2-ylmethyl, and -CH₂-CH₂-N(CH₃)₂.

In one embodiment, R¹ and R² are selected from a optionally substituted heteroaryl, -CH₃, -C₂H₅, - C₃H₇, -C₄H₉, C₆H₁₃, C₈H₁₇, C₁₂H₂₅, C₁₈H₃₇, and -CH₂-CH₂-N(CH₃)₂. More preferably, at least one of R¹ and R² is pyridin-2-yl, optionally substituted by -C₁₋₁₂alkyl or benzyl. Even more preferably, at least one of R¹ or R² is pyridin-2-ylmethyl and the other is selected from -CH₃, -C₂H₅, -C₃H₇, -C₄H₉, -C₆H₁₃, -C₈H₁₇, -C₁₂H₂₅.

X¹ is preferably selected from -C=O.

Preferably, R³ and R⁴ are equal and are selected from -C(O)-O-CH₃, -C(O)-O- CH₂CH₃, -C(O)-O-CH₂C₆H₅ and -CH₂OH.

Preferably, at least one of R¹ or R² is pyridin-2-ylmethyl and the other is selected from -CH₃, -C₂H₅, - C₃H₇, -C₄H₉, -C₆H₁₃, -C₈H₁₇, -C₁₂H_{25;} R³ and R⁴ are equal and are selected from -C(O)-O-CH₃, -C(O)-O- CH₂CH₃, -C(O)-O-CH₂C₆H₅ and -CH₂OH; and X¹ is selected from -C=O, and R⁶ and R⁷ are H.

Preferably, at least one of R¹ or R² is pyridin-2-ylmethyl and the other is selected from -CH₃, -C₂H₅, - C₃H₇, -C₄H₉, -C₆H₁₃, -C₈H₁₇, -C₁₂H_{25;} R³ and R⁴ are equal and are selected from -C(O)-O-CH₃, -C(O)-O- CH₂CH₃, and -C(O)-O-CH₂C₆H₅; and X is -C=O, and R⁶ and R⁷ are H.

A preferred class of bispidon is one in which at least one of R¹ or R² is pyridin-2-ylmethyl or benzyl, preferably pyridin-2-ylmethyl. More preferably, R¹ is pyridin-2-ylmethyl and R² is methyl.

A preferred ligand is dimethyl 2,4-di-(2-pyridyl)-3-methyl-7-(pyridin-2-ylmethyl)-3,7-diaza-bicyclo[3.3.1]nonan-9-one-1,5-dicarboxylate (N2py3o-C1) and the iron complex thereof [Fe(N2py3o-C1)CI]CI which can be prepared as described in WO 02/48301. Another suitable ligand is dimethyl 2,4-di-(2-pyridyl)-3-methyl-7-(N,N-dimethyl-amino-ethyl)-3,7-diaza-bicyclo[3.3.1]nonan-9-one-1,5-dicarboxylate and the iron complex thereof. Other preferred bispidons are ones in which instead of having a methyl group at the 3 position have longer alkyl chains, namely isobutyl, (n-hexyl) C6, (n-octyl) C8, (n-dodecyl) C12, (n-tetradecyl) C14, (n-octadecyl) C18, which were prepared in an analogous manner. Preferred tetradentate bispidons are also described in WO00/60045 and preferred pentadentate bispidons are described in WO02/48301 and WO03/104379.

Preferably the iron ion is selected from Fe(II) and Fe(III), preferably the iron ion is Fe(II).

In a preferred embodiment, the at least one nitrogen donor ligand in a) is selected from the compounds of formula (II) or (III). In a more preferred embodiment, the nitrogen donor ligand in a) is the compound of formula (II).

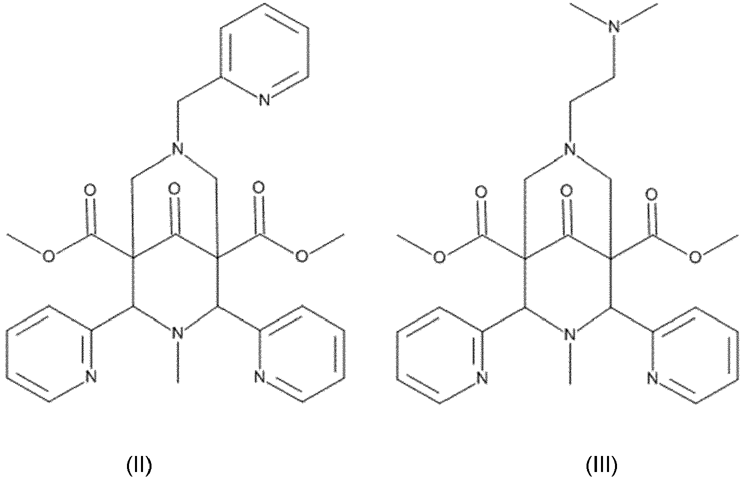

Preferably the ligand L is present in one or more of the forms [FeLCl₂], [FeLCI]CI, [FeL(H₂O)](PF₆)₂, [FeL]Cl₂, [FeLCl]PF₆, and [FeL(H₂O)](BF₄)₂. Preferably the ligand L is present in one or more of the form [FeLCl₂], [FeLCI]CI, [FeL]Cl₂ and [FeL(H₂O)](BF₄)₂.

Preferably, the iron complex in a) is a compound of formula (IV): 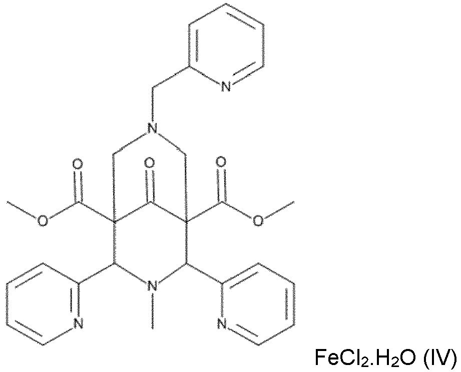 which may also be referred to as the active ingredient of Borchi^{®} Oxy Coat from OMG with CAS nr. 478945-46-9.

In some embodiments, the iron complex is the complex defined as CAS nr. 478945-46-9.

In some embodiments, the iron complex is iron (1+),chloro[dimethyl-9,9-dihydroxy-3-methyl-2,4-di-(2-pyridyl-kN)-7-[(2-pyridinyl-kN)methyl]-3,7-diazabicyclo[3.3.1]nonane-1,5-dicarboxylate-kN3,kN7]-,chloride(1-).

The drier composition according to the first aspect of the invention also comprises:
b) at least one metal salt of a carboxylic acid, wherein the metal is selected from the group comprising: Mn, Ce, V, and Cu.

In a preferred embodiment, the metal in b) is selected from Mn or Ce. In a preferred embodiment, the metal in b) is Mn.

In a preferred embodiment, the carboxylic acid is selected from branched-chain or straight-chain saturated and unsaturated aliphatic, aromatic and alicyclic monocarboxylic acids having 5 to 22 carbon atoms, cycloaliphatic monocarboxylic acids having 5 to 10 carbon atoms, or mixtures of these acids. Preferably the carboxylic acid is selected from the group comprising 2-ethylbutanoic acid, 2,2-dimethylpentanoic acid, 2-ethylpentanoic acid, 2-ethyl-4-methylpentanoic acid, 2-ethylhexanoic acid, isooctanoic acid, isononanoic acid, neononanoic acid, nonanoic acid, isodecanoic acid, neodecanoic acid, 2-ethyldecanoic acid, isotridecanoic acid, isotetradecanoic acid, n-hexanoic acid, n-octanoic acid, n-decanoic acid, n-dodecanoic acid, cyclopentanoic acid, methylcyclopentanoic acid, cyclohexanoic acid, methylcyclohexanoic acid, 1,2-dimethylcyclohexanoic acid, cycloheptanoic acid, myristic acid, stearic acid, arachidic acid, behenic acid, oleic acid, linoleic acid, tall oil fatty acid, erucic acid, p-tert-butylbenzoicacid, monobutyl maleate, monodecyl phthalate, naphthenic acid and mixtures thereof. In a preferred embodiment, the carboxylic acid is 2-ethylhexanoic acid, neodecanoic acid, or naphthenic acid.

In an embodiment, the at least one Mn salt of a carboxylic acid is a polymer compound comprising a manganese-bearing polymer, said polymer compound having a manganese content of 0.5 % to 12 % by weight, a mean molecular weight of more than 2000, comprising manganese carboxylate sequences, with an acid value of less than 40 mg KOH /g, preferably of less than 20 mg KOH /g. Fatty acids are the preferred carboxylic acids, as such alkyd type polymers are more compatible with the alkyd binders used in paints and inks. For the same reason, the polymer could have an excess of hydroxyl groups. The mean molecular weight can be estimated from the remaining free functionalities of the polymer, or by any appropriate analytical technique. The polymer compound has preferably a manganese content of between 1 and 6 % by weight. Also, a mean molecular weight of more than 3000 is preferred. It may be unsaturated. Polymer compounds having a mean molecular weight of less than 250000 are preferred. The manganese atoms can be an integral part of the backbone chain of the polymer. By this is meant that the manganese atoms form links in the backbone chain of polymers. The process for preparing such polymer compound can comprise the steps of reacting a manganese salt or oxide, preferably manganese acetate, with an acid functional polymer, or with a mixture of polybasic and monobasic carboxylic acids, thereby obtaining a first intermediate compound; and, of reacting said first intermediate compound with a polyol, or a mixture of polyols, until water evolution ceases. Suitable divalent carboxylic acids are dimeric fatty acids, orthophtalic acid, isophtalic acid, terephtalic acid, maleic acid, adipic acid, succinic acid, sebacic acid, dodecanoic acid, or any mixture thereof. Optionally, monobasic acids are added, such as a saturated or unsaturated fatty acid after reaction with dienes, neodecanoic acid, naphthenic acid, isononanoic acid, stearic acid, or any mixture thereof. Suitable polyols are glycerol, pentaerythritol, dipentaerythritol, synthetic polyols, or any mixture thereof. The reactants are reacted in such proportions that there is preferably a small excess of basic constituents, which results is a limited amount of free hydroxyl functionality. This tends to insure a good solubility in the binder formulation. Generally, but not exclusively, the manganese compound is first reacted with an excess of the carboxylic acids, resulting in a lower molecular weight manganese bearing compound with excess carboxylic functionality, which is then further reacted with one or more polyols to the final macro molecular structure. Preferred polymer compounds are described in WO2012000934.

The drier composition according to the first aspect of the invention also comprises as component (c) at least one ligand comprising at least one moiety selected from the group comprising 1,4,7-tri-azacyclononanyl, 2,2'-bipyridyl, 1,10-phenantrolinyl, imidazolyl, pyrazolyl, porphyrinyl, aliphatic, cycloaliphatic, and aromatic amino.

In some embodiments, the at least one ligand comprises an imidazole. As used herein, the term "imidazole" refers to a compound with the formula C₃H N₂. Imidazole refers to the parent compound whereas imidazoles are a class of heterocycles that share the 1,3-C₃N₂ ring but with varying substituents. Non-limiting examples of suitable imidazole ligands are selected from the group comprising: 1-octyl-1H-imidazole, 2-ethyl-4-methylimidazole, N, N-bis- (2-ethyl-4-methylimidazol-5-ylmethyl) aminopropane, 1,1'-(1,3-propanediyl)bis(1H-imidazole), 1,1'-(1,4-butatanediyl)bis(1H-imidazole), 1,1'-(1,5-pentanediyl)bis(1H-imidazole), 1,1'-(1,6-hexanediyl)bis(1H-imidazole), and 1,1'-[1,4-phenylenebis(methylene)]bis(1H-imidazole).

In some embodiments, the at least one ligand comprises a pyrazole. As used herein, the term "pyrazole" refers to a compound with the formula C₃H₃N₂H. Pyrazole refers to the parent compound whereas pyrazoles are the class of compounds that have the ring C₃N₂ with adjacent nitrogen centers. Non-limiting examples of suitable imidazole ligands are selected from the group comprising: 3-phenyl-1H-pyrazole, 3-(4-methoxyphenyl)pyrazole, 3,3'-(1,3-phenylene)bis(1H-pyrazole), and 3,3'-(1,4-phenylene)bis(1H-pyrazole).

In some embodiments, the at least one ligand comprises an amine. As used herein, the term "amine" refers to compounds that contain a basic nitrogen atom with a lone pair. The term "amine" comprises primary, secondary, and tertiary amines. Non-limiting examples of suitable imidazole ligands are selected from the group comprising: ethylenediamine, N,N'-tetramethylethylenediamine, diethylenetriamine, 1,1,4,7,7-pentamethyldiethylenetriamine triethylenetetramine, 1,1,4,7,10,10-hexamethyltriethylenetetramine, tetraethylenepentamine, pentaethylenehexamine, and N,N'-tetramethylpropylenediamine.

As used herein, the term "ligand comprising at least one 1,4,7-tri-azacyclononanyl moiety" encompasses ligands which comprise at least one optionally substituted 1,4,7-triazacyclononane structure which has one or more nitrogen groups that complex with one metal salt of a carboxylic acid, to provide a tridentate, tetradentate, pentadentate or hexadentate ligand.

In a preferred embodiment, the at least one ligand in c) is a compound of formula (V), wherein 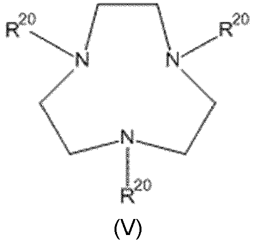
each R²⁰ is independently selected from the group consisting of C₁₋₁₀alkyl, C₃₋₈cycloalkyl, heterocycloalkyl, heteroaryl, C₆₋₁₀aryl and C₆₋₁₀aryl-C₁₋₆alkyl, C₁₋₆alkyl-C₆₋₁₀aryl, each group being optionally substituted with one or more substituents each independently selected from the group consisting of-OH, C₁₋₆alkoxy, phenoxy, carboxylate, carboxamide, carboxylic ester, sulfonate, amine, C₁₋₆alkylamino and -N⁺(R²¹)₃;
each R²¹ is independently selected from -H, C₁₋₁₀alkyl, C₂₋₁₀alkenyl, C₆₋₁₀aryl-C₁₋₆alkyl, C₆₋₁₀aryl-C₂₋₆alkenyl, C₁₋₆alkyloxy, C₂₋₆alkenyloxy, aminoC₁₋₆alkyl, aminoC₂₋₆alkenyl, C₂₋₂₄alkyl ether, C₃₋₂₄alkenyl ether, and -CX²₂-R²²; and,
each X² is independently selected from -H or C₁₋₃alkyl; and wherein each R²² is independently selected from an optionally C₁₋₆alkyl-substituted heteroaryl group selected from the group consisting of pyridyl, pyrazinyl, pyrazolyl, pyrrolyl, imidazolyl, benzimidazolyl, pyrimidinyl, triazolyl and thiazolyl; preferably wherein at least one of R²¹ is -C(X²)₂-R²².

In some embodiments, each R²⁰ is independently selected from the group consisting of C₁₋₆alkyl, C₃₋₈cycloalkyl, heterocycloalkyl, heteroaryl, C₆₋₁₀aryl and C₆₋₁₀aryl-C₁₋₆alkyl, C₁₋₆alkyl-C₆₋₁₀aryl, each group being optionally substituted with one or more substituents each independently selected from the group consisting of -OH, C₁₋₆alkoxy, phenoxy, carboxylate, carboxamide, carboxylic ester, sulfonate, amine, C₁₋₆alkylamine and -N⁺(R²¹)₃;
each R²¹ is independently selected from -H, C₁₋₆alkyl, C₂₋₆alkenyl, C₆₋₁₀aryl-C₁₋₆alkyl, C₆₋₁₀aryl-C₂₋₆alkenyl, C₁₋₆alkyloxy, C₂₋₆alkenyloxy, aminoC₁₋₆alkyl, aminoC₂₋₆alkenyl, C₂₋₆alkyl ether, C₃₋₆alkenyl ether, and -CX²₂-R²²; and,
each X² is independently selected from -H or C₁₋₃alkyl; and wherein each R²² is selected from an optionally C₁₋₄alkyl-substituted heteroaryl group selected from the group consisting of pyridyl, pyrazinyl, pyrazolyl, pyrrolyl, imidazolyl, benzimidazolyl, pyrimidinyl, triazolyl and thiazolyl; wherein at least one of R²¹ is -C(X²)₂-R²².

In some embodiments, each R²⁰ is independently selected from the group consisting of C₁₋₄alkyl, C₃₋₈cycloalkyl, heterocycloalkyl, heteroaryl, C₆₋₁₀aryl and C₆₋₁₀aryl-C₁₋₄alkyl, C₁₋₄alkyl-C₆₋₁₀aryl, each group being optionally substituted with one or more substituents each independently selected from the group consisting of -OH, C₁₋₄alkoxy, phenoxy, carboxylate, carboxamide, carboxylic ester, sulfonate, amine, C₁₋₄alkylamine and -N⁺(R²¹)₃;
each R²¹ is independently selected from -H, C₁₋₄alkyl, C₂₋₆alkenyl, C₆₋₁₀aryl-C₁₋₄alkyl, C₆₋₁₀aryl-C₂₋₆alkenyl, C₁₋₄alkyloxy, C₂₋₆alkenyloxy, aminoC₁₋₄alkyl, aminoC₂₋₆alkenyl, C₂₋₄alkyl ether, C₃₋₆alkenyl ether, and -CX²₂-R²²; and,
each X² is independently selected from -H or C₁₋₃alkyl; and wherein each R²² is an independently selected organic group, preferably independently selected from an optionally C₁₋₆alkyl-substituted heteroaryl group selected from the group consisting of pyridyl, pyrazinyl, pyrazolyl, pyrrolyl, imidazolyl, benzimidazolyl, pyrimidinyl, triazolyl and thiazolyl; wherein at least one of R²¹ is -C(X²)₂-R²².

In a preferred embodiment, the at least one organic compound in (c) is a compound of formula (XI), wherein
each R²⁰ is independently selected from the group consisting of C₁₋₄alkyl, C₃₋₆cycloalkyl, heterocycloalkyl, heteroaryl, C₆₋₁₀aryl and C₆₋₁₀aryl-C₁₋₄alkyl, optionally substituted with a substituent selected from the group consisting of-OH, C₁₋₄alkoxy, phenoxy, carboxylate, carboxamide, carboxylic ester, sulfonate, amine, C₁₋₄alkylamine and -N⁺(R²¹)₃;
each R²¹ is selected from -H, C₁₄alkyl, C₂₋₄alkenyl, C₆₋₁₀aryl-C₁₋₄alkyl, C₆₋₁₀aryl-C₂₋₄alkenyl, C₁₋₄alkyloxy, C₂₋₄alkenyloxy, aminoC₁₋₄alkyl, aminoC₂₋₄alkenyl, C₂₋₄alkyl ether, C₃₋₄alkenyl ether, and-CX²₂-R²²;
each X² is independently selected from -H or C₁₋₃alkyl; and wherein each R²² is independently selected from an optionally C₁₋₄alkyl-substituted heteroaryl group selected from the group consisting of pyridyl, pyrazinyl, pyrazolyl, pyrrolyl, imidazolyl, benzimidazolyl, pyrimidinyl, triazolyl and thiazolyl; and, wherein at least one of R²¹ is -C(X²)₂-R²². Preferably R²² is selected from optionally substituted pyridin-2-yl, imidazol-4-yl, pyrazol-1-yl, quinolin-2-yl groups. Most preferably R²² is either a pyridin-2-yl or a quinolin-2-yl.

The ligand of formula (V) is also referred to as TACN-Nx.

Preferably, the ligand in c) is 1,4,7-trimethyl-1,4,7-tri-azacyclononane.

Preferably the ligand is a porphyrin ligand. A porphyrin is a compound containing four nitrogen heterocycles arranged in a cyclic structure. For example, in some embodiments, the porphyrin ligands of interest have the structure of Formula (VI) 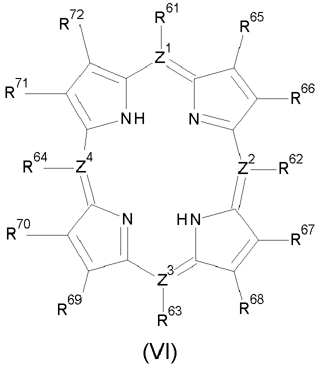
Z¹, Z², Z³, and Z⁴ are independently selected from C and N;
R⁶¹-R⁷² are each independently selected from hydrogen, halo, C₁₋₂₄alkyl, C₂₋₂₄alkenyl, C₂₋₂₄alkynyl, C₆₋₂₀aryl, C₆₋₂₄alkaryl, C₆₋₂₄aralkyl, hydroxyl, C₁₋₂₄alkoxy, C₂₋₂₄alkenyloxy, C₂₋₂₄alkynyloxy, C₆₋₂₀aryloxy, acyloxy, acyl, C₂₋₂₄alkoxycarbonyl, C₆₋₂₀aryloxycarbonyl, C₁₋₂₄alkylcarbonyl, C₆₋₂₀arylcarbonyl, halocarbonyl, formyl, thioformyl, C₁₋₂₄alkylcarbonato, C₆₋₂₀arylcarbonato, carboxy, carboxylato, carbamoyl, thiocarbamoyl, carbamato, carbamido, cyano, isocyano, cyanato, isocyanato, isothiocyanato, amino, C₁₋₂₄alkylamido, C₆₋₂₀arylamido, imino, alkylimino, arylimino, nitro, nitroso, sulfhydryl, C₁₋₂₄alkylsulfanyl, C₆₋₂₀arylsulfanyl, sulfo, sulfino, sulfonyl, phosphino, phosphono, and O-phosphono, provided that when Z¹, Z², Z³, and Z⁴ is N, then the corresponding R group (R⁶¹, R⁶², R⁶³, or R⁶⁴, respectively) is not present. Any such groups may be unsubstituted or substituted and may contain one or more heteroatoms as appropriate (i.e., as the chemical nature of the group allows for such substitution or heteroatoms). Furthermore, any two adjacent groups selected from R⁶¹-R⁷² may be taken together to form a cycle, wherein such cycle may be aliphatic, aromatic, heteroatom-containing, and/or substituted as appropriate. It will be appreciated that the dashed lines and double bonds shown in the formulae herein are drawn in certain orientations but are not intended to imply a definite or fixed location of such bonds. In other words, resonance structures of the formulae drawn herein are intended to be within the scope of the invention.

For example, in some embodiments, Z¹, Z², Z³, and Z⁴ are N, and R⁶¹, R⁶², R⁶³, and R⁶⁴ are not present. In some embodiments, Z¹, Z², Z³, and Z⁴ are C, and R⁶¹, R⁶², R⁶³, and R⁶⁴ are present. In some embodiments, one or more of Z¹, Z², Z³, and Z⁴ are C, and one or more of Z¹, Z², Z³, and Z⁴ are N.

For example, R⁶¹-R⁷² are independently selected from: hydrogen; halo, including F, Cl, Br, and I; substituted or unsubstituted C₁₋₂₄alkyl, C₂₋₂₄alkenyl, C₂₋₂₄alkynyl, C₆₋₂₀aryl, C₆₋₂₄alkaryl, C₆₋₂₄aralkyl; substituted or unsubstituted heteroatom-containing C₁₋₂₄alkyl, C₂₋₂₄alkenyl, C₂₋₂₄alkynyl, C₆₋₂₀aryl, C₆₋₂₄alkaryl, C₆₋₂₄aralkyl; hydroxyl; substituted or unsubstituted C₁₋₂₄alkoxy, C₂₋₂₄alkenyloxy, C₂₋₂₄alkynyloxy, C₆₋₂₀aryloxy, acyloxy, acyl, C₁₋₂₄alkoxycarbonyl, C₆₋₂₀aryloxycarbonyl, C₁₋₂₄alkylcarbonyl, C₆₋₂₀arylcarbonyl, halocarbonyl, formyl, thioformyl; C₁₋₂₄alkylcarbonato, C₆₋₂₀arylcarbonato; carboxy and carboxylato (including C₁₋₂₄alkylcarboxylato and C₆₋₂₀arylcarboxylato); carbamoyl (including mono-(C₁₋₂₄alkyl)-substituted carbamoyl, di-(C₁₋₂₄alkyl)-substituted carbamoyl, mono-substituted arylcarbamoyl, and mixed alkyl/aryl substituted carbamoyl) and thiocarbamoyl; carbamato (including mono-(C₁₋₂₄alkyl)-substituted carbamato, di-(C₁₋₂₄alkyl)-substituted carbamato, mono-substituted arylcarbamato, and mixed alkyl/aryl substituted carbamato); carbamido, cyano, isocyano, cyanato, isocyanato, and isothiocyanato; amino (including mono- and di-(C₁₋₂₄alkyl)-substituted amino, mono- and di-(C₆₋₂₀aryl)-substituted amino, and mixed alkyl/aryl substituted amino); alkylamido and C₆₋₂₀arylamido; imino, alkylimino, and arylimino; nitro; nitroso; sulfhydryl (including C₁₋₂₄alkylsulfanyl, and C₆₋₂₀arylsulfanyl); sulfo (including C₁₋₂₄alkylsulfonato, and C₆₋₂₀arylsulfonato); sulfino (including C₁₋₂₄alkylsulfinyl, and C₆₋₂₀arylsulfinyl); sulfonyl (including C₁₋₂₄alkylsulfonyl, and C₆₋₂₀arylsulfonyl); phosphino (including mono-, di-, and tri-(C₁₋₂₄alkyl)-substituted phosphinato, mono-, di-, and tri-(C₆₋₂₀aryl)-substituted phosphinato, mixed alkyl/aryl substituted phosphinato, and phosphine oxides); and phosphono (including mono- and di-(C₁₋₂₄alkyl)-substituted phosphonato, mono- and di-(C₆₋₂₀aryl)-substituted phosphonato, mixed alkyl/aryl substituted phosphonato, and O-phosphonato).

R⁶¹-R⁷² may be selected from enols, ketones, esters, aldehydes, anhydrides, and acyl halides, ethers, epoxies, phosphonics, phosphates, phospinites, phosphate esters, imides, azides, azoles, nitrates, nitriles, carbimides, aziridines, hydroxylamines, ketoximes, aldoximes, nitrate esters, enamines, azoles, imidazols, pyrroles, indoles, purines, pyrimidines, piperidines, pyridazines, pyridyl and derivatives, linear, cyclic and aromatic, oxyhalides, sulfides, thioethers, thioesters, sulfonates, sulfinyls, thiocyanates, disulfides, sulfones, thioamides, sulfoxides, isothyocyanates, sulfonamides, sulfonyl halides, thioureates, and thiophosphate esters.

In some embodiments, R⁶¹, R⁶², R⁶³, and R⁶⁴ are the same. For example, in some embodiments, R⁶¹, R⁶², R⁶³, and R⁶⁴ are the same and are selected from hydrogen, substituted or unsubstituted C₁₋₂₄alkyl, substituted or unsubstituted heteroatom-containing C₁₋₂₄alkyl, substituted or unsubstituted C₆₋₂₀aryl, and substituted or unsubstituted heteroaryl. For example, R⁶¹, R⁶², R⁶³, and R⁶⁴ are the same and are selected from hydrogen, phenyl, and methoxyphenyl.

In some embodiments, R⁶⁵, R⁶⁶, R⁶⁷, R⁶⁸, R⁶⁹, R⁷⁰, R⁷¹, and R⁷² are the same. For example, in some embodiments, R⁶⁵, R⁶⁶, R⁶⁷, R⁶⁸, R⁶⁹, R⁷⁰, R⁷¹, and R⁷² are the same and are hydrogen.

In some embodiments, R⁶¹, R⁶², R⁶³, and R⁶⁴ are the same and are a first group, R⁶⁵, R⁶⁶, R⁶⁷, R⁶⁸, R⁶⁹, R⁷⁰, R⁷¹, and R⁷² are the same and are a second group, and the first and second groups are different groups.

In some embodiments, Z¹, Z², Z³, and Z⁴ are the same. In other embodiments, Z¹, Z², Z³, and Z⁴ are not the same.

In some embodiments, the ligands of interest have the structure of Formula (VII): 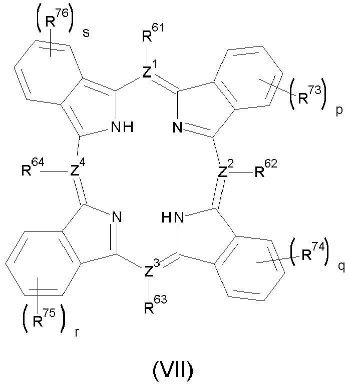 wherein Z¹, Z², Z³, and Z⁴, R⁶¹, R⁶², R⁶³, and R⁶⁴ are as defined in formula (VI) above; p, q, r, and s are independently selected from the integers 0, 1, 2, 3, and 4; and each R⁷³, R⁷⁴, R⁷⁵, and R⁷⁶ is independently selected from the groups defined above for R⁶¹-R⁷² in formula (V).

For example, in some embodiments, p, q, r, and s are each 0.

In some embodiments, pairs of substituents selected from R⁷³, R⁷⁴, R⁷⁵, and R⁷⁶ may be taken together to form further cycles, wherein such cycles are aliphatic, aromatic, heteroatom-containing, and/or substituted.

For example, in some embodiments, the ligand is phthalocyanine, tetrabenzoporphyrin, tetraazaporphyrin, or porphyrin.

Ligands of interest include tetraarylporphyrins, diarylporphyrins, tetraalkylporphyrins, dialkylporphyrins, and mixed aryl/alkyl porphyrins, as well as porphyrins containing alkenyl substituents, alkynyl substituents, heteroatom-containing substituents (e.g., heteroaryl, etc.), functionalized substituents (e.g., alkyl substituted with a carboxyl group, etc.), and the like. Specific ligands of interest include, but are not limited to: phthalocyanine; tetrabenzoporphyrin; tetraazaporphyrin; tetratolylporphyrin; porphyrin; porphyrazine; 5,10,15,20-tetrakisphenylporphyrin; 5,10,15,20-tetrakis(4'-methoxyphenyl)porphyrin; 5-azaprotoporphyrin dimethylester; bis-porphyrin; coproporphyrin III; coproporphyrin III tetramethylester; deuteroporphyrin; deuteroporphyrin IX dimethylester; diformyldeuteroporphyrin IX dimethyl ester, dodecaphenylporphyrin; hematoporphyrin; hematoporphyrin IX; hematoporphyrin monomer; hematoporphyrin dimer; hematoporphyrin derivative; hematoporphyrin IX dimethylester; haematoporphyrin IX dimethylester; mesoporphyrin dimethylester; mesoporphyrin IX dimethylester; monoformyl-monovinyl-deuteroporphyrin IX dimethylester; monohydroxyethylvinyl deuteroporphyrin; 5,10,15,20-tetra(o-hydroxyphenyl)porphyrin; 5,10,15,20-tetra(m-hydroxyphenyl)porphyrin; 5,10,15,20-tetrakis-(m-hydroxyphenyl)porphyrin; 5,10,15,20-tetra(p-hydroxyphenyl)porphyrin; 5,10,15,20-tetrakis-(3-methoxyphenyl)porphyrin; 5,10,15,20-tetrakis-(3,4-dimethoxyphenyl)porphyrin; 5,10,15,20-tetrakis(3,5-dimethoxyphenyl)porphyrin; 5,10,15,20-tetrakis-(3,4,5-trimethoxyphenyl)porphyrin; 2,3,7,8,12,13,17,18-octaethyl-5,10,15,20-tetraphenylporphyrin; Photofrin; porphyrin c; protoporphyrin; protoporphyrin IX; protoporphyrin dimethylester; protoporphyrin IX dimethylester; protoporphyrin propylaminoethylformamide iodide; protoporphyrin N,N-dimethylaminopropylformamide; protoporphyrin propylaminopropylformamide iodide; protoporphyrin butylforinamide; protoporphyrin N<∼>-dimethylamino-formamide; protoporphyrin formamide; sapphyrin 13,12,13,22-tetraethyl-2,7,18,23 tetramethyl sapphyrin-8,17-dipropanol; sapphyrin 2 3,12,13,22-tetraethyl-2,7,18,23 tetramethyl sapphyrin-8-monoglycoside; sapphyrin 3; meso-tetra-(4-N-carboxyphenyl)-porphine; tetra-(3-methoxyphenyl)-porphine; tetra-(3-methoxy-2,4-difluorophenyl)-porphine; 5,10,15,20-tetrakis(4-N-methylpyridyl)porphine; meso-tetra-(4-N-methylpyridyl)porphine tetrachloride; meso-tetra(4-N-methylpyridyl)porphine; meso-tetra-(3-N-methylpyridyl)-porphine; meso-tetra-(2-N-methylpyridyl)porphine; tetra(4-NNN-trimethylanifinium)porphine; meso-tetra-(4-NNN"-trimethylamino-phenyl)porphine tetrachloride; tetranaphthaloporphyrin; 5,10,15,20-tetraphenylporphyrin; tetraphenylporphyrin; meso-tetra-(4-N-sulfonatophenyl)-porphine; tetraphenylporphine tetrasulfonate; meso-tetra-(4-sulfonatophenyl)porphine; tetra-(4-sulfonatophenyl)porphine; tetraphenylporphyrin sulfonate; meso-tetra-(4-sulfonatophenyl)porphine; tetrakis-(4-sulfonatophenyl)porphyrin; meso-tetra(4-sulfonatophenyl)porphine; meso-(4-sulfonatophenyl)porphine; meso-tetra-(4-sulfonatophenyl)porphine; tetrakis(4-sulfonatophenyl)porphyrin; meso-tetra-(4-N-trimethylanilinium)-porphine; uroporphyrin; uroporphyrin I; uroporphyrin IX; and uroporphyrin III. In some embodiments, naturally derived porphyrins are suitable, such as the porphyrin ligands found in heme or chlorophyll. Additional specific ligands and methods for their preparation can be found in the relevant literature, such as Kadish et al., Handbook of Porphyrin Science: With Applications to Chemistry, Physics, and Materials (World Scientific, 2010).

Preferably the components (b) and (c) of the drier composition can be provided as complexes.

In a preferred embodiment, the drier composition comprises one or more carboxylate salt of compound of formula (VIII), as a complex of (b) and (c).

In a preferred embodiment, the drier composition comprises the 2-ethylhexanoate and acetate salt of a compound of formula (VIII), as a complex of (b) and (c). 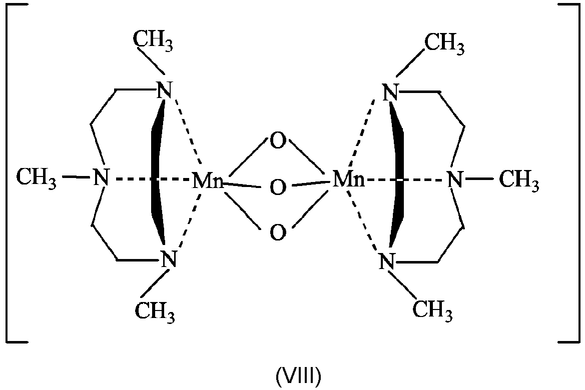 which may also be referred to as the active ingredient of Drycoat.

Preferably the components (b) and (c) of the drier composition can be provided as a complex, wherein said complex comprise the reaction mass of tri-µ-(2-ethylhexanoato-O)-bis(N,N',N"-trimethyl-1,4,7-triazacyclononane-N,N',N")di-manganese and µ-(acetato-O)-di-µ-(2-ethylhexanoato-O)-bis(N,N',N"-trimethyl-1,4,7-triazacyclononane-N,N',N")di-manganese (Cas 1381939-25-8, EG-nr.: 937-913-7).

In some preferred embodiments, the drier composition comprises a manganese compound as defined in WO 2014/086556, preferably a manganese compound according to paragraphs [026]-[029] of WO 2014/086556.

In some preferred embodiments, the drier composition comprises one or more compounds of general structure (I*) or mixtures of different compounds of general structure (I*): 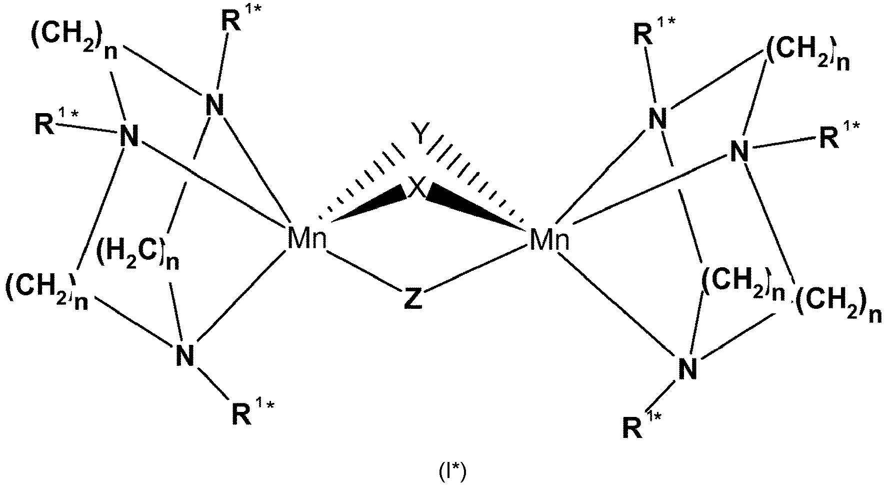
wherein each R^{1*} are identical or different from each other, and independently selected from the group consisting of C₁₋₁₈-alkyl, C₁₋₁₈-alkenyl, C₁₋₁₈-alkynyl, and C₃₋₁₂-cycloalkyl; preferably wherein each R^{1*} are identical or different from each other and independently selected from the group consisting of C_{1- 18}-alkyl; more preferably wherein each R^{1*} is methyl;
wherein n is an integer in a range between 1 and 5, preferably between 2 and 5, for example 1, 2, 3, 4, or 5, more preferably wherein n is 2;
wherein X, Y, and Z are identical or different from each other, and are R^{2*}COO⁻; and wherein each R^{2*} are identical or different from each other, and independently selected from the group consisting of H, C₁₋₁₈-alkyl, C₁₋₁₈-alkenyl, C₁₋₁₈-alkynyl, C₃₋₁₂-cycloalkyl, C₃₋₁₂-cycloalkenyl, C₃₋₁₂-heterocycloalkyl, C₇₋₁₂-aralkyl; preferably wherein each R^{2*} are identical or different from each other and independently selected from C₁₋₁₈-alkyl.

In some preferred embodiments, the drier composition comprises one or more compounds of general structure (I*) or mixtures of different compounds of general structure (I*);
wherein each R^{1*} are identical or different from each other, and independently selected from the group consisting of C₁₋₁₈-alkyl;
wherein n is an integer in a range between 1 and 5, preferably between 2 and 5, for example 1, 2, 3, 4, or 5, more preferably wherein n is 2;
wherein X, Y, and Z are identical or different from each other, and are R^{2*}COO⁻;
wherein each R^{2*} are identical or different from each other and independently selected from the group consisting of C₁₋₁₈-alkyl.

In some preferred embodiments, the drier composition comprises one or more compounds of general structure (II*) or mixtures of different compounds of general structure (II*): 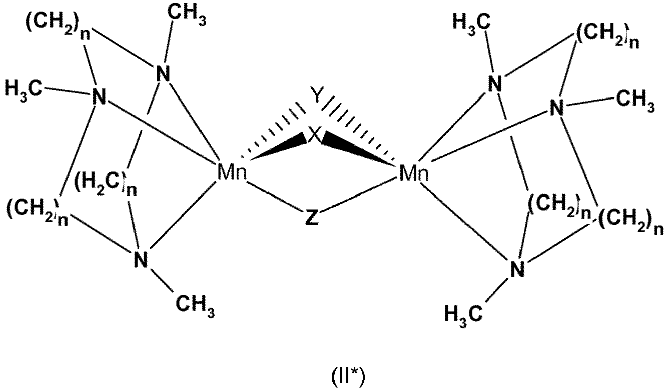
wherein n is an integer in a range between 1 and 4, preferably between 2 and 4, for example 1, 2, 3, or 4, more preferably wherein n is 2 or 3;
wherein X, Y, and Z are identical or different from each other, and are selected from the group consisting of CH₃-COO⁻ and CH₃-(CH₂)₃-CH(CH₃CH₂)-COO⁻.

In some preferred embodiments, the drier composition comprises the compound with CAS Number [1381939-25-8]. In some preferred embodiments, the drier composition comprises the compound with EG number 937-913-7. In some preferred embodiments, the drier composition comprises the compound with the Reach Registration Number 01-2119919049-35-0000.

In some preferred embodiment, components (b) and (c) of the drier composition are provided as the complex defined by CAS 1381939-25-8. In some preferred embodiment, components (b) and (c) of the drier composition are provided as the complex defined by EG 937-913-7. In some preferred embodiment, components (b) and (c) of the drier composition are provided as the complex defined by Reach Registration Number 01-2119919049-35-0000.

In some preferred embodiments, the drier composition comprises a composition comprising <5% 2-ethyl-hexanol (CAS: 104-76-7, EG: 203-234-3), <10% Drycoat- Manganese complex (CAS: 1381939-25-8, EG: 937-913-7, registration number: 01-2119919049-35-0000), and 70-90% dearomatised kerosene (CAS: 64742-48-9, EG: 265-150-3, registration number: 01-2119457273-39).

Preferably the components (b) and (c) of the drier composition can be provided as a complex, wherein said complex comprises manganese 2-ethylhexanoate and 2,2'-bipyridyl.

Preferably the components (b) and (c) of the drier composition can be provided as a complex, wherein said complex comprises manganese 2-ethylhexanoate and tolyldiethanolamine.

Accordingly, in some embodiments the complexes of interest are carboxylic salts of structures of Formula (IX) or (X)

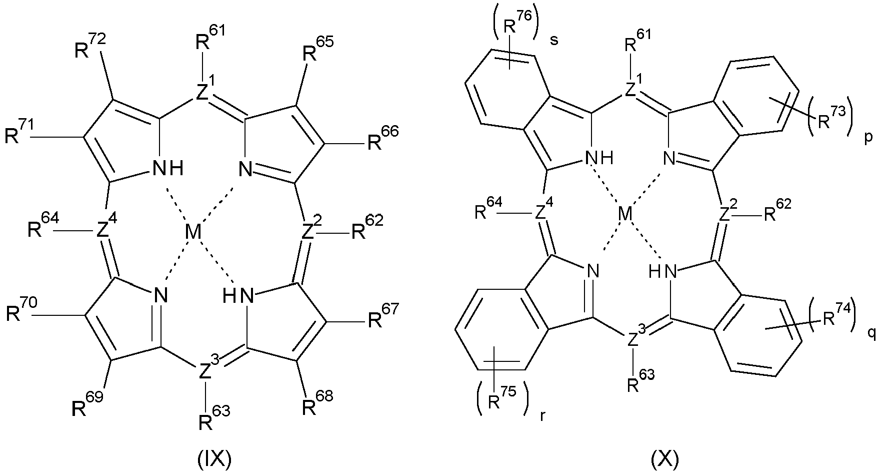

Wherein M is selected from Mn, Ce, V, and Cu; and p, q, r, s, Z¹- Z⁴ and R⁶¹-R⁷⁶ are as described above in Formula (VI) and Formula (VII).

Preferably the components (b) and (c) of the drier composition can be provided as a complex, wherein said complex comprises manganese octoate and a porphyrin ligand.

In some embodiments, the drier composition comprises:
a) at least one Fe complex comprising Fe and at least one nitrogen donor ligand, wherein said at least nitrogen donor ligand is selected from the group comprising tetradentate, pentadentate and hexadentate nitrogen donor ligands, preferably wherein the ligand is a compound of formula (I) ;
b) at least one metal salt of a carboxylic acid, wherein the metal is Mn; and
c) at least one ligand comprising at least one moiety selected from the group comprising 1,4,7-tri-azacyclononanyl, 2,2'-bipyridyl, 1,10-phenantrolinyl, porphyrinyl; preferably at least one ligand comprising at least one 1,4,7-tri-azacyclononanyl; preferably the ligand is 1,4,7-trimethyl-1,4,7-tri-azacyclononane.

In some embodiments, the drier composition comprises:
a) at least one Fe complex comprising Fe and at least one nitrogen donor ligand of formula (I);
   wherein R¹ and R² are each independently selected from the group consisting of C₁₋₂₄alkyl, C₆₋₁₀aryl, heteroaryl, heteroarylC₁₋₆alkyl, and C₁₋₆alkylaminoC₁₋₆alkyl; wherein heteroaryl is selected from the group consisting of pyridyl, pyrazinyl, and pyrazolyl ;
   R³ and R⁴ are each independently selected from the group consisting of-H, C₁₋₈alkyl, C₁₋₈alkyl-O-C₁₋₈alkyl, C₁₋₈alkyl-O-C₆₋₁₀aryl, C₆₋₁₀aryl, hydroxyC₁₋₈alkyl, and -(CH₂)ₘC(O)OR⁵; R⁵ is selected from -H, C₁₋₄alkyl, C₆₋₁₀aryl, or C₁₋₄alkylC₆₋₁₀aryl, and m is an integer selected from 0, 1, 2, 3, or 4;
   each R⁶ and R⁷ are independently selected from the group consisting of-H, -F, -CI, -Br, -OH, C₁₋₄alkoxy, -NH-C(O)-H, -NH-C(O)-C₁₋₄alkyl, -NH₂, -NH-C₁₋₄alkyl, and C₁₋₄alkyl; and,
   X¹ is selected from -C(O)- or -[C(R⁸)₂]ₙ-; wherein n is an integer selected from 0, 1, 2 or 3, and each R⁸ is independently selected from the group consisting of-H, -OH, C₁₋₄alkoxy and C₁₋₄alkyl;
b) at least one metal salt of a carboxylic acid, wherein the metal is Mn; and
c) at least one ligand comprising at least one moiety selected from the group comprising 1,4,7-tri-azacyclononanyl, 2,2'-bipyridyl, 1,10-phenantrolinyl, porphyrinyl, preferably at least one ligand comprising at least one 1,4,7-tri-azacyclononanyl; preferably the ligand is 1,4,7-trimethyl-1,4,7-tri-azacyclononane.

In some embodiments, the drier composition comprises:
a) at least one Fe complex comprising Fe and at least one nitrogen donor ligands, having the structural formula (II) or (III), preferably (II);
b) at least one metal salt of a carboxylic acid, wherein the metal is Mn; and
c) at least one ligand comprising at least one moiety selected from the group comprising 1,4,7-tri-azacyclononanyl, 2,2'-bipyridyl, 1,10-phenantrolinyl, porphyrinyl, preferably at least one ligand comprising at least one 1,4,7-tri-azacyclononanyl; preferably the ligand is 1,4,7-trimethyl-1,4,7-tri-azacyclononane.

In some embodiments, the drier composition comprises:
a) at least one Fe complex comprising Fe and at least one nitrogen donor ligand of formula (II) or (III), preferably (II); and
b-c) at least one Mn carboxylate complex of formula (VIII).

In some embodiments, the drier composition comprises:
a) at least one Fe complex comprising Fe and at least one nitrogen donor ligand of formula (II) or (III), preferably (II); and
b-c) at least one Mn carboxylate complex of formula (I*).

In some embodiments, the drier composition comprises:
a) at least one Fe complex comprising Fe and at least one nitrogen donor ligand of formula (II) or (III), preferably (II); and
b-c) at least one Mn carboxylate complex of formula (II*).

In some embodiments, the drier composition comprises:
a) at least one Fe complex comprising Fe and at least one nitrogen donor ligand of formula (II) or (III), preferably (II); and
b-c) at least one complex defined by CAS 1381939-25-8.

In some embodiments, the drier composition comprises:
a) at least one Fe complex of formula (IV);
b) at least one metal salt of a carboxylic acid, wherein the metal is Mn; and
c) at least one ligand comprising at least one moiety selected from the group comprising 1,4,7-tri-azacyclononanyl, 2,2'-bipyridyl, 1,10-phenantrolinyl, porphyrinyl, preferably at least one ligand comprising at least one 1,4,7-tri-azacyclononanyl; preferably the ligand is 1,4,7-trimethyl-1,4,7-tri-azacyclononane.

In some embodiments, the drier composition comprises:
a) at least one Fe complex of formula (IV); and
b-c) at least one Mn carboxylate complex of formula (VIII).

In some embodiments, the drier composition comprises:
a) at least one Fe complex of formula (IV); and
b-c) at least one Mn carboxylate complex of formula (I*).

In some embodiments, the drier composition comprises:
a) at least one Fe complex of formula (IV); and
b-c) at least one Mn carboxylate complex of formula (II*).

In some embodiments, the drier composition comprises:
a) at least one Fe complex of formula (IV); and
b-c) at least one complex defined by CAS 1381939-25-8.

According to an embodiment, the drier composition also comprises at least one potassium (K) salt of a carboxylic acid.

Preferably, the carboxylic acid of said at least one K salt of a carboxylic acid is selected from branched-chain or straight-chain saturated and unsaturated aliphatic, aromatic and alicyclic monocarboxylic acids having 5 to 22 carbon atoms, cycloaliphatic monocarboxylic acids having 5 to 10 carbon atoms, or mixtures of these acids, preferably the carboxylic acid is selected from the group comprising 2-ethylbutanoic acid, 2,2-dimethylpentanoic acid, 2-ethylpentanoic acid, 2-ethyl-4-methylpentanoic acid, 2-ethylhexanoic acid, isooctanoic acid, isononanoic acid, neononanoic acid, nonanoic acid, isodecanoic acid, neodecanoic acid, 2-ethyldecanoic acid, isotridecanoic acid, isotetradecanoic acid, n-hexanoic acid, n-octanoic acid, n-decanoic acid, n-dodecanoic acid, cyclopentanoic acid, methylcyclopentanoic acid, cyclohexanoic acid, methylcyclohexanoic acid, 1,2-dimethylcyclohexanoic acid, cycloheptanoic acid, myristic acid, stearic acid, arachidic acid, behenic acid, oleic acid, linoleic acid, tall oil fatty acid, erucic acid, p-tert-butylbenzoic acid, monobutyl maleate, monodecyl phthalate, naphthenic acid and mixtures thereof. Particularly preferred acids include 2-ethylhexanoic acid, isononanoic acid, isodecanoic acid, and mixtures thereof. Preferably said K salt of a carboxylic acid is selected from the group comprising K-octoate, K-naphthenate and K-neodecanoate. More preferably, said K salt of a carboxylic acid is K-octoate.

Suitable potassium (K) driers are available from the OM Group, Inc., and include Potassium Hex-Cem^{®}.

In some embodiments, the drier composition also comprises at least one Ca, Zr, Sr, Zn, Ba, Li, or Bi salt of an organic acid, preferably of a carboxylic acid.

In some embodiments, the drier composition also comprises at least one Ca salt of an organic acid. Examples of suitable calcium (Ca) salts of an organic acid include, but are not limited to: calcium carboxylates such as calcium neodecanoates, calcium octoates, calcium tallates, calcium linoleates, calcium propionate, calcium decanate, calcium isononanoate , and calcium naphthenates. Such calcium (Ca) driers are available from the OM Group, Inc., and include calcium Ten-Cem^{®}, calcium Cem-All^{®}, calcium Hex-Cem^{®}, and calcium Nap-All.

In some embodiments, the drier composition also comprises at least one Zr salt of an organic acid. Examples of suitable zirconium (Zr) salts of an organic acid include, but are not limited to: zirconium carboxylates such as zirconium propionate, zirconium neodecanoates, zirconium octoates, and zirconium naphthenates and mixtures thereof. Such zirconium (Zr) driers are available from the OM Group, Inc., and include zirconium Hex-Cem^{®}.

Examples of suitable strontium (Sr) salts of an organic acid include, but are not limited to: strontium carboxylates such as strontium octoate. Such strontium driers are available from the OM Group, Inc., and include strontium Hex-Cem^{®}.

According to a second aspect, the present invention also relates to a coating composition, comprising:
a) at least one autoxidizable alkyd based binder; and
b) a drier composition according to the first aspect of the invention, and preferred embodiments thereof.

According to the invention, the coating composition comprises at least one autoxidizable alkyd binder.

As used herein, the term "alkyd binder" or "alkyd resin" are used interchangeably. Suitable autoxidizable alkyd resin for use in the invention, are in general the reaction product of the esterification of polyhydric alcohols with polybasic acids (or their anhydrides) and unsaturated fatty acids (or glycerol esters thereof), for example derived from linseed oil, tung oil, tall oil as well as from other drying or semi-drying oils. Alkyd resins are well-known in the art and need not to be further described herein. The properties are primarily determined by the nature and the ratios of the alcohols and acids used and by the degree of condensation. Suitable alkyds include long oil, very long oil and medium oil alkyd resins e.g. derived from 45 to 85 wt% of fatty acids, preferably derived from 45 to 70 wt% of fatty acids. As used herein, the term "long oil alkyd" refers to alkyd with an oil content of between 60 and 75 wt%, and a fatty acid content of 57 to 70 wt%. As used herein, the term "medium oil alkyd " refers to alkyd with an oil content of between 45 wt% and 60 wt%, and a fatty acid content of 42 to 57 wt%. As used herein, the term "very long oil alkyd" refers to alkyd with an oil content of between 75 and 85 wt%, and a fatty acid content of 70 to 80 wt%. In an embodiment, the alkyd is a medium oil alkyd. In another embodiment, the alkyd is a long oil alkyd. In another embodiment, the alkyd is a very long oil alkyd. To improve the performance of the resins, the composition of the long oil and medium oil alkyd may be modified. For example, polyurethane modified alkyds, silicone modified alkyds, styrene modified alkyds, acrylic modified alkyds (e.g. (meth)acrylic modified alkyds), vinylated alkyds, polyamide modified alkyds, and epoxy modified alkyds or mixtures thereof are also suitable alkyd resins to be used in the present composition.

Preferably, said at least one autoxidizable alkyd binder is selected from a medium or long oil unmodified alkyd, a silicone modified alkyd, a polyurethane modified alkyd, acrylic modified alkyd, polyamide modified alkyd, or a combination thereof. Most preferably, said alkyd binder is a long oil (unmodified) alkyd, a silicone modified alkyd, a polyurethane modified alkyd, acrylic modified alkyd, polyamide modified alkyd, or a combination thereof.

The amount of alkyd binder in the present compositions can typically range from about 10 to 98 % by weight, such as about 15 to about 90 % by weight, preferably about 20 to 80 % by weight, preferably about 25 to 70 % by weight based on the total weight of the composition.

In a preferred embodiment of the present invention, the alkyd binder has a solids content of at least 50%, preferably at least 55%, more preferably at least 60% yet more preferably at least 65%, yet more preferably at least 70%, whereby the solids content is defined as non-volatile solids content or non-volatile matter or nvm. As used herein, the term "solids content" refers to the proportion of non-volatile material contained in an adhesive, coating, ink, paint, or other suspension. It is the material left after the volatile solvent (which serves as a carrier or vehicle for the solid content) has vaporized. The solids content may be determined by evaporating to dryness a weighed sample of solution and determining the percent residue. More details on how the solids content may be measured can be found in ISO3251.

In certain embodiments, the coating composition of the present invention further comprises anti-skinning agents and anti-oxidants such as but not limited to methyl ethyl ketoxime, n-butyl ketoxime, cyclohexanone oxime, methyl isobutyl ketoxime, acetone oxime, 2-cyclohexylphenol, 4-cyclohexylphenol, mono-tertiary butylhydroquinone, diethyl hydroxylamine, 2-[(1-methylpropyl)amino]ethanol, and 2,4-pentadione and the like.

In a preferred embodiment, the coating composition further comprises an oxime.

In a preferred embodiment, the coating composition does not include any additional anti-skinning agents, or includes only limited amounts of additional anti-skinning agents. For example, the coating composition may comprise at most 0.01% by weight of anti-skinning agent other than an oxime, preferably at most 0.001% by weight, preferably at most 0.0001% by weight, based on the total weight of the coating composition. Preferably the coating composition is essentially free of anti-skinning agents other than an oxime. Suitable oximes are such as but not limited to: formaldehyde oxime, acetaldehyde oxime, propionaldehyde oxime, acetone oxime, butyraldehyde oxime, isobutyraldehyde oxime, methyl ethyl ketoxime, 2,3-butanedione monoxime, 4-methylpentan-2-one oxime, 3-methylbutyraldehyde oxime, 2,4-dimethylpentan-3-one oxime, cyclopentanone oxime, hexan-2-one oxime, cyclohexanone oxime, 3-pyridinealdoxime, 4-pyridinealdoxime, heptanal oxime, 5-methylhexan-2-one oxime, benzaldehyde oxime, salicylaldoxime, acetophenone oxime, and benzophenone oxime. In some embodiments, the coating composition may comprise at most 0.01% by weight of anti-skinning agent other than methyl ethyl ketoxime, preferably at most 0.001% by weight, preferably at most 0.0001% by weight, based on the total weight of the coating composition. Preferably the coating composition is essentially free of anti-skinning agents other than methyl ethyl ketoxime.

In an embodiment, the composition comprises at least 0.0001 % to at most 1 % by weight of the Fe in a), preferably at least 0.00010 % to at most 0.1 % by weight of the Fe in a), more preferably at least 0.00015 % to at most 0.01 % by weight of the Fe in a), for example at least 0.0002 to at most 0.002 % by weight of the Fe in a),considering only the amount of Fe of said at least one Fe complex, with weight percent being based on the total weight of the alkyd binder.

In an embodiment, the composition comprises at least 0.0001 % to at most 2 % by weight of the metal in b), preferably at least 0.0002 % to at most 0.1 % by weight of the metal in b), more preferably at least 0.0005 % to at most 0.05 % by weight of the metal in b), for example at least 0.001 % to at most 0.02 % by weight of the metal in b), considering only the amount of metal of said at least one metal salt of a carboxylic acid, with weight percent being based on the total weight of the alkyd binder.

In an embodiment, the coating composition comprises at least 0.001% and at most 0.06% by weight of the metal in b), based on the total weight of the alkyd binder. For example, the coating composition may comprise at most 0.05% by weight of the metal in b), for example at most 0 0.04% by weight of the metal in b), for example at most 0.03% by weight of the metal in b), based on the total weight of the alkyd binder.

In an embodiment, the composition comprises from 0.0001 % to 2 % by weight metal, preferably from 0.0002 % to 1.5 % by weight metal, more preferably from 0.0005 % to 1.0 % by weight metal, with weight percent being based on the total weight of the alkyd binder, wherein all metal of the composition is included, which comprises the Fe of the Fe complex and the metal of the least one metal salt of a carboxylic acid as well as the metal of optional additional driers.

In some embodiments, the drier composition comprises:
a) at least one Fe complex comprising Fe and at least one nitrogen donor ligand, wherein said at least nitrogen donor ligand is selected from the group comprising tetradentate, pentadentate and hexadentate nitrogen donor ligands, preferably wherein the ligand is a compound of formula (I); with at least 0.0001 % to at most 1 % by weight of the Fe in a), preferably at least 0.00010 % to at most 0.1 % by weight of the Fe in a), more preferably at least 0.00015 to at most 0.01 % by weight of the Fe in a), for example at least 0.0002 % to at most 0.002 % by weight of the Fe in a), considering only the amount of Fe of said at least one Fe complex, with weight percent being based on the total weight of the alkyd binder;
b) at least one metal salt of a carboxylic acid, wherein the metal is Mn; with at least 0.0001 % to at most 2 % by weight of the metal in b), preferably at least 0.0002 % to at most 0.1 % by weight of the metal in b), more preferably at least 0.0005 % to at most 0.05 % by weight of the metal in b), for example at least 0.001 % to at most 0.02 % by weight of the metal in b), considering only the amount of Mn of said at least one metal salt of a carboxylic acid, with weight percent being based on the total weight of the alkyd binder; and
c) at least one ligand comprising at least one moiety selected from the group comprising 1,4,7-tri-azacyclononanyl, 2,2'-bipyridyl, 1,10-phenantrolinyl, porphyrinyl; preferably at least one ligand comprising at least one 1,4,7-tri-azacyclononanyl; preferably the ligand is 1,4,7-trimethyl-1,4,7-tri-azacyclononane.

In some embodiments, the drier composition comprises:
a) at least one Fe complex comprising Fe and at least one nitrogen donor ligand of formula (I); with at least 0.0001 % to at most 1 % by weight of the Fe in a), preferably at least 0.00010 % to at most 0.1 % by weight of the Fe in a), more preferably at least 0.00015 % to at most 0.01 % by weight of the Fe in a), for example at least 0.0002 % to at most 0.002 % by weight of the Fe in a),considering only the amount of Fe of said at least one Fe complex, with weight percent being based on the total weight of the alkyd binder;
   wherein R¹ and R² are each independently selected from the group consisting of C₁₋₂₄alkyl, C₆₋₁₀aryl, heteroaryl, heteroarylC₁₋₆alkyl, and C₁₋₆alkylaminoC₁₋₆alkyl; wherein heteroaryl is selected from the group consisting of pyridyl, pyrazinyl, and pyrazolyl ;
   R³ and R⁴ are each independently selected from the group consisting of-H, C₁₋₈alkyl, C₁₋₈alkyl-O-C₁₋₈alkyl, C₁₋₈alkyl-O-C₆₋₁₀aryl, C₆₋₁₀aryl, hydroxyC₁₋₈alkyl, and -(CH₂)ₘC(O)OR⁵; R⁵ is selected from -H, C₁₋₄alkyl, C₆₋₁₀aryl, or C₁₋₄alkylC₆₋₁₀aryl, and m is an integer selected from 0, 1, 2, 3, or 4;
   each R⁶ and R⁷ are independently selected from the group consisting of-H, -F, -CI, -Br, -OH, C₁₋₄alkoxy, -NH-C(O)-H, -NH-C(O)-C₁₋₄alkyl, -NH₂, -NH-C₁₋₄alkyl, and C₁₋₄alkyl; and,
   X¹ is selected from -C(O)- or -[C(R⁸)₂]ₙ-; wherein n is an integer selected from 0, 1, 2 or 3, and each R⁸ is independently selected from the group consisting of-H, -OH, C₁₋₄alkoxy and C₁₋₄alkyl;
b) at least one metal salt of a carboxylic acid, wherein the metal is Mn; with at least 0.0001 % to at most 2 % by weight of the metal in b), preferably at least 0.0002 % to at most 0.1 % by weight of the metal in b), more preferably at least 0.0005 % to at most 0.05 % by weight of the metal in b), for example at least 0.001 % to at most 0.02 % by weight of the metal in b), considering only the amount of metal of said at least one metal salt of a carboxylic acid, with weight percent being based on the total weight of the alkyd binder; and
c) at least one ligand comprising at least one moiety selected from the group comprising 1,4,7-tri-azacyclononanyl, 2,2'-bipyridyl, 1,10-phenantrolinyl, porphyrinyl, preferably at least one ligand comprising at least one 1,4,7-tri-azacyclononanyl; preferably the ligand is 1,4,7-trimethyl-1,4,7-tri-azacyclononane.

In some embodiments, the drier composition comprises:
a) at least one Fe complex comprising Fe and at least one nitrogen donor ligands, having the structural formula (II) or (III), preferably (II); with at least 0.0001 % to at most 1 % by weight of the Fe in a), preferably at least 0.00010 % to at most 0.1 % by weight of the Fe in a), more preferably at least 0.00015 % to at most 0.01 % by weight of the Fe in a), for example at least 0.0002 % to at most 0.002 % by weight of the Fe in a),considering only the amount of Fe of said at least one Fe complex, with weight percent being based on the total weight of the alkyd binder;
b) at least one metal salt of a carboxylic acid, wherein the metal is Mn; with at least 0.0001 % to at most 2 % by weight of the metal in b), preferably at least 0.0002 % to at most 0.1 % by weight of the metal in b), more preferably at least 0.0005 % to at most 0.05 % by weight of the metal in b), for example at least 0.001 % to at most 0.02 % by weight of the metal in b), considering only the amount of metal of said at least one metal salt of a carboxylic acid, with weight percent being based on the total weight of the alkyd binder; and
c) at least one ligand comprising at least one moiety selected from the group comprising 1,4,7-tri-azacyclononanyl, 2,2'-bipyridyl, 1,10-phenantrolinyl, porphyrinyl, preferably at least one ligand comprising at least one 1,4,7-tri-azacyclononanyl; preferably the ligand is 1,4,7-trimethyl-1,4,7-tri-azacyclononane.

In some embodiments, the drier composition comprises:
a) at least one Fe complex comprising Fe and at least one nitrogen donor ligand of formula (II) or (III), preferably (II); with at least 0.0001 % to at most 1 % by weight of the Fe in a), preferably at least 0.00010 % to at most 0.1 % by weight of the Fe in a), more preferably at least 0.00015 % to at most 0.01 % by weight of the Fe in a), for example at least 0.0002 % to at most 0.002 % by weight of the Fe in a),considering only the amount of Fe of said at least one Fe complex, with weight percent being based on the total weight of the alkyd binder; and
b-c) at least one Mn carboxylate complex of formula (VIII); with at least 0.0001 % to at most 2 % by weight of the metal in b), preferably at least 0.0002 % to at most 0.1 % by weight of the metal in b), more preferably at least 0.0005 % to at most 0.05 % by weight of the metal in b), for example at least 0.001 % to at most 0.02 % by weight of the metal in b), considering only the amount of metal of said at least one metal salt of a carboxylic acid, with weight percent being based on the total weight of the alkyd binder.

In some embodiments, the drier composition comprises:
a) at least one Fe complex of formula (IV); and at least 0.0001 % to at most 1 % by weight of the Fe in a), preferably at least 0.00010 % to at most 0.1 % by weight of the Fe in a), more preferably at least 0.00015 % to at most 0.01 % by weight of the Fe in a), for example at least 0.0002 % to at most 0.002 % by weight of the Fe in a),considering only the amount of Fe of said at least one Fe complex, with weight percent being based on the total weight of the alkyd binder;
b) at least one metal salt of a carboxylic acid, wherein the metal is Mn; with at least 0.0001 % to at most 2 % by weight of the metal in b), preferably at least 0.0002 % to at most 0.1 % by weight of the metal in b), more preferably at least 0.0005 % to at most 0.05 % by weight of the metal in b), for example at least 0.001 % to at most 0.02 % by weight of the metal in b), considering only the amount of metal of said at least one metal salt of a carboxylic acid, with weight percent being based on the total weight of the alkyd binder; and
c) at least one ligand comprising at least one moiety selected from the group comprising 1,4,7-tri-azacyclononanyl, 2,2'-bipyridyl, 1,10-phenantrolinyl, porphyrinyl, preferably at least one ligand comprising at least one 1,4,7-tri-azacyclononanyl; preferably the ligand is 1,4,7-trimethyl-1,4,7-tri-azacyclononane.

In some embodiments, the drier composition comprises:
a) at least one Fe complex of formula (IV); and at least 0.0001 % to at most 1 % by weight of the Fe in a), preferably at least 0.00010 % to at most 0.1 % by weight of the Fe in a), more preferably at least 0.00015 % to at most 0.01 % by weight of the Fe in a), for example at least 0.0002 % to at most 0.002 % by weight of the Fe in a),considering only the amount of Fe of said at least one Fe complex, with weight percent being based on the total weight of the alkyd binder; and
b-c) at least one Mn carboxylate complex of formula (VIII); with at least 0.0001 % to at most 2 % by weight of the metal in b), preferably at least 0.0002 % to at most 0.1 % by weight of the metal in b), more preferably at least 0.0005 % to at most 0.05 % by weight of the metal in b), for example at least 0.001 % to at most 0.02 % by weight of the metal in b), considering only the amount of metal of said at least one metal salt of a carboxylic acid, with weight percent being based on the total weight of the alkyd binder.

In an embodiment, said composition can be formulated as an emulsion, preferably an alkyd emulsion. Alkyd emulsions can be made in a variety of different methods. In an embodiment, said composition additionally comprises water and an alkyd emulsion is obtained by phase inversion or homogenisation.

Despite the choice of emulsion technology/process the outcome is dispersed alkyd droplets in a water phase, wherein the water is the continuous media of the system. Emulsifiers can be used. A combination of two or more different surfactants may be used, such as a combination of one ionic (small molecule that possesses high diffusion rate) for promoting the drops during the formation and one nonionic (large polymeric surfactant) for stabilizing the droplets on a long term perspective. Alternatively stable alkyd emulsions can be produced in the absence of an external emulsifier by neutralizing the free carboxyl groups of the alkyd resin with an amine. After neutralization alkyd resins can be emulsified directly in water without use of low molecular weight emulsifiers. The shearing device can be for instance a dissolver, specially designed stirrer, rotor-stator homogenizator or high pressure homogenizator. A typical solid content of an alkyd emulsion is from 30-60%.

Preferably, the coating composition is a solvent-borne coating composition.

As used herein, the term "solvent-borne coating composition" refers to a composition that utilizes one or more volatile organic materials as the primary dispersing medium. According to certain embodiments, the coating compositions of the present invention can be substantially free of water, or, in some cases, completely free of water.

As used herein, the term "volatile organic material" refers to any organic compound having an initial boiling point less than or equal to 250°C measured at a standard pressure of 101.3 kPa.

As used herein, the term "organic compound" refers to any compound containing at least the element carbon and one or more of hydrogen, oxygen, sulfur, phosphorus, silicon, nitrogen, or a halogen, with the exception of carbon oxides and inorganic carbonates and bicarbonates.

Volatile organic materials are often included in coating compositions to reduce the viscosity of the composition sufficiently to enable forces available in simple coating techniques, such as spraying, to spread the coating to controllable, desired and uniform thicknesses. Also, volatile organic materials may assist in substrate wetting, resinous component compatibility, package stability and film formation. Non-limiting examples of suitable volatile organic materials (also referred as solvent) for use in the present composition include aliphatic, cycloaliphatic, aromatic hydrocarbons and oxygenated solvents, such as hexane, heptane, octane, isooctane, cyclohexane, cycloheptane, toluene and xylene; isoparafins; ketones, such as methyl ethyl ketone and methyl isobutyl ketone; alcohols, such as isopropyl alcohol, normal-butyl alcohol and normal-propyl alcohol; monoethers of glycols, such as the monoethers of ethylene glycol and diethylene glycol; di-ethers of glycols such as dipropylene glycol dimethyl ether; monoether glycol acetates, such as 2-ethoxyethyl acetate; as well as compatible mixtures thereof. As examples of such solvents may be mentioned hydrocarbon solvents available under the trademarks Shellsol H, Shellsol K, Shellsol D40, Shellsol D60, Shellsol D70, and Shellsol AB, all from Shell Chemicals, the Netherlands, the trademarked Solvesso 150 solvent from Esso and also: Exxsol D40, Exxsol D60 and Exxsol D80, and solvents such as ethyl diglycol, ethyl glycol acetate, butyl glycol, butyl glycol acetate, butyl diglycol, butyl diglycol acetate, and methoxypropylene glycol acetate. Preferably the solvent is an aliphatic hydrocarbon solvent, preferably a high-boiling aliphatic hydrocarbon solvent, such as Shellsol D40 and D60. (The boiling point range for Shellsol D40 is 163 - 196 °C and for D60: 177-213 °C).

As used herein, the term "substantially free" means that the material being discussed is present in the composition, if at all, as an incidental impurity. In other words, the material does not affect the properties of the composition. As used herein, the term "completely free" means that the material being discussed is not present in the composition at all.

In certain embodiments, the amount of water present in the coating compositions of the present invention is less than 25 % by weight, such as less than 20 % by weight, such as less than 15 % by weight, such as less than 10 % by weight, such as less than 5 % by weight, or, in some cases, less than 2 % by weight, or, in yet other cases, less than 1 % by weight, with the % by weight being based on the total weight of the coating composition. The amount of water should remain lower than 25 % by weight such that the alkyd binder remains in the continuous phase.

In a preferred embodiment, the coating composition further comprises at least one organic solvent in an amount of about 0.1 % by weight to about 50 % by weight, preferably about 1.0 % by weight to about 45 % by weight, preferably about 5 % by weight to about 40 % by weight, preferably about 10 % by weight to about 35 % by weight, preferably about 15 % by weight to about 30 % by weight, based on the total weight of the coating composition.

In certain embodiments, the coating compositions of the present invention comprise at least one colorant. The colorant component of the coating composition may comprise one or more inorganic or organic, transparent or non-transparent pigments. Non-limiting examples of such pigments are titanium dioxide, iron oxides, mixed metal oxides, bismuth vanadate, chromium oxide green, ultramarine blue, carbon black, lampblack, monoazo and disazo pigments, anthraquinones, isoindolinones, isoindolines, quinophthalones, phthalocyanine blues and greens, dioxazines, quinacridones and diketo-pyrrolopyrroles; and extender pigments including ground and crystalline silica, barium sulfate, magnesium silicate, calcium silicate, mica, micaceous iron oxide, calcium carbonate, zinc oxide, aluminum hydroxide, aluminum silicate and aluminum silicate, gypsum, feldspar, talcum, kaolin, and the like. The amount of pigment that is used to form the coating composition is understood to vary, depending on the particular composition application, and can be zero when a clear composition is desired.

For example, a coating composition may comprise up to about 300 % by weight, for example about 0 to about 200 % by weight of pigment based on the solids content of the alkyd resin (pigment/binder), preferably up to 100 % by weight of pigment based on the solids content of the alkyd resin. Depending on the particular end use, a preferred composition may comprise approximately 0 to 100 % by weight of pigment based on the solids content of the alkyd resin.

The coating compositions of the present invention may include other additives, e.g. catalysts, other pigments and pigment pastes, dyes, fillers, stabilizers, thixotropic agents, anti-sagging agents, anti-oxidants, antifouling agents, anti-gelling agents, bactericides, fungicides, algaecides, insecticides, anti-settling agents, antifoaming agents, slip agents, flow and leveling agents, rheological modifiers, photo-initiators, UV-absorbers, synergists, HALS-radical scavengers, corrosion inhibitors, matting agents, waxes, mineral oils, flame retardants, anti-static agents, loss of dry inhibitors, optical brighteners, adhesion promoters, diluents, elastomers, plasticizers, air release agents, desiccants, anti-crater additives, reinforcing agents, dispersing aids, substrate wetting agents, odorants, corrosion-inhibitive pigments, additional hardeners and additional curable compounds, depending on the application. Certain embodiments of the coating compositions of the present invention include surface active agents, such as any of the well known anionic, cationic or nonionic surfactants or dispersing agents. Examples of suitable additives that may be added to the coating composition may be found in Additives Guide, Paint & Coatings Magazine, May 2006. If desired, other resinous materials can be utilized in conjunction with the aforementioned alkyd resins.

The metal drier combinations and optionally colorants, pigments and extenders and optionally other additives may be formulated into the coating compositions by mixing and, if appropriate, dispersing and grinding with the liquid binder.

In a preferred embodiment of the invention, the coating composition is formulated as a one package coating composition, also referred herein as one-component (1K) coating composition.

A "1K" or "one package" composition will be understood as referring to a composition wherein all of the components are maintained in the same container after manufacture, during storage, etc. A "two packages" or "2K" composition will be understood as referring to a composition wherein two components are maintained separately until just prior to application. A "multi packages" or "multicomponents" composition will be understood as referring to a composition wherein various components are maintained separately until just prior to application.

The coating composition according to the invention can be used and/or formulated as varnish, lacquer, paint, stain, enamel, printing ink or floor covering and similar compositions which contain autoxidizable alkyd binders.

According to a third aspect, the present invention also relates to the use of the coating composition according to the second aspect of the invention in a varnish, lacquer, paint, stain, enamel, printing ink or floor covering.

According to a fourth aspect, the present invention also relates to a substrate having applied thereon a coating composition according to the second aspect of the invention.

The coating compositions of the present invention can be applied to various substrates including wood, paper, foam, and synthetic materials (such as plastics including elastomeric substrates), leather, textiles, glass, ceramic, metals (such as iron, steel and aluminum), concrete, cement, brick, and the like.

As a result, the present invention is also directed to substrates at least partially coated with at least one coating composition of the present invention. The substrates may be pretreated before application of the at least one coating composition. The substrates may be post-treated after application of the at least one coating composition, with any other compositions.

Any known method can be used to apply the coating compositions of the invention to a substrate. Non-limiting examples of such application methods are spreading (e.g., with paint pad or doctor blade, or by brushing or rolling), spraying (e.g., air-fed spray, airless spray, hot spray, and electrostatic spray), flow coating (e.g., dipping, curtain coating, roller coating, and reverse roller coating), and electrodeposition. (See generally, R. Lambourne, Editor, Paint and Surface Coating: Theory and Practice, Eilis Horwood, 1987, page 39 et seq.).

The coating compositions of the present invention can be applied and fully cured at ambient temperature conditions in the range of from about -10°C to 50°C. Curing of said polymer composition according to the invention typically can proceed very rapidly, and in general can take place at a temperature within the range of from -10 °C to +50 °C, in particular from 0 °C to 40 °C, more in particular from 3 to 25 °C. However, compositions of the present invention may be cured by additional heating.

The coating compositions of the present invention may be used as a single coating, a top coating, a base coating in a two-layered system, or one or more layers of a multi-layered system including a clear top coating composition, colorant layer and base coating composition, or as a primer layer. A typical opaque system may comprise: 1 or 2 layers primer and 1 or 2 layers of top coat (a total of 3 layers). Alternative opaque systems may comprise: 1 primer layer, 1 layer of mid coat and 1 layer top coat. Examples of transparent systems may comprise 1 layer of impregnant and 3 layers of top coats or 3 layers of top coat for maintenance work.

The invention will be more readily understood by reference to the following examples, which are included merely for purpose of illustration of certain aspects and embodiments of the present invention and are not intended to limit the invention.

### EXAMPLES

Several examples and comparative examples are described hereunder illustrating the effect of the compositions according to embodiments of the present invention on the drying and skinning properties.

Unless otherwise indicated, all parts and all percentages in the following examples, as well as throughout the specification, are parts by weight or percentages by weight respectively.

Drying times were assessed in the following manner:
- The test composition was cast on a glass plate by using a draw bar with a gap size of 100 µm.
- Dust-free: The coating is considered dust-free if it does not pull fibers when a wad of cotton is blown gently of a drying film in a horizontal direction.
- Tack-free: The coating is considered tack free if it does not pull fibers when a wad of cotton is placed on the drying film with a load of 1 kg/3 cm2 for 10 seconds and afterwards blown gently away in a horizontal direction.

Drying times were obtained under ambient conditions of 23°C and 50% RH and under adverse drying conditions of 5°C and 85% RH.

The storage stability tests were performed in closed jars filled to 40% of the volume and having an air packed head space of 60%. The jars containing the paint products were stored at 23 °C and examined at specified time intervals for signs of skin formation. A skin on the surface of the composition was considered to have formed when it had sufficient tensile strength to support a gentle indentation by a spatula. Anti-skinning performance was defined as the number of days at which skin formation was first observed.

Discoloration is inversely proportional to the percentage of whiteness in the color of the dried coat and percentage whiteness can be conveniently measured by conventional spectrophotometric techniques. Therefore percentage whiteness is a convenient inverse measure of discoloration. To measure the initial whiteness and the yellowing of the coating film, the test formulations were applied to prepared color cards by using a draw bar with a gap size of 250 µm. The films were allowed to dry under controlled conditions of constant temperature and humidity for 24 hours. The relative whiteness of a paint film was measured by conventional spectrophotometric techniques according to the Cie-lab algorithm. After 2 weeks of storing in daylight, these color cards were placed in an oven under conditions of 50 °C and the air fully saturated with water vapor for 24 hours and were measured again.

A typical base paint without metal driers was prepared by mixing together the constituents listed in Table 1.

**Table 1**

| constituents | parts by weight |
|---|---|
| long oil alkyd | 26.4 |
| polyurethane modified alkyd | 38.2 |
| acrylic modified alkyd | 10.6 |
| high boiling aliphatic hydrocarbon solvent | 3.8 |
| defoamer | 0.7 |
| calcium (5%) drier | 2.2 |
| zirconium (18%) drier | 2.2 |
| white alkyd colorant | 9.9 |
| black alkyd colorant | 6.0 |
| total | 100.0 |

The solids content of the long oil alkyd was 90% nvm, the solids content of the polyurethane modified alkyd was 75% nvm and the solids content of acrylic modified alkyd was 85% (nvm). The total solids binder content of the base paint in Table 1 was 68.4 wt% (including the alkyd resins in the black and white colorant).
zirconium (18%) drier: siccative based on zirconium 2-ethylhexanoate and having a zirconium content of 18%
calcium (5%) drier: siccative based on calcium isononanoate and having a calcium content of 5%

An enamel was prepared by mixing together the constituents listed in Table 1 with a drier composition according to the invention or with a comparative drier composition, as shown in Table 2.

Following compounds were used:
- Methyl ethyl ketoxime, also abbreviated as MEKO. The MEKO commercially available from Rockwood under the tradename Exkin II and the methyl ethyl ketoxime content was > 99%.
- Exkin OXF: MEKO-free antiskinning agent, comprising diethylhydroxylamine, triphenyl phosphite and strontium carboxylate, commercially available from Rockwood.
- Ascinin^{®} Anti Skin 0444: MEKO-free antiskinning agent, comprising an amino compound, commercially available from OMG.
- Cobalt (10%) drier: siccative based on cobalt neodecanoate and having a cobalt content of 10%, commercially available from OMG-Borchers as Borchers^{®} Deca Cobalt 10.
- Borchi^{®} OXY-Coat: contains 1 wt% Fe-complex (bispidon ligand), equivalent to 0,09 wt% Fe, dissolved in propylene glycol (99 wt%, EINECS 200-33-8-0), commercially available from OMG, comprising the complex of formula (IV) (CAS nr. 478945-46-9, also known as iron (1+),chloro[dimethyl-9,9-dihydroxy-3-methyl-2,4-di-(2-pyridyl-kN)-7-[(2-pyridinyl-kN)methyl]-3,7-diazabicyclo[3.3.1]nonane-1,5-dicarboxylate-kN3,kN7]-,chloride(1-)).
- Borchi^{®} OXY-Coat 1101: contains 1 wt% Fe-complex (bispidon ligand), equivalent to 0.09 wt% Fe, dissolved in water, commercially available from OMG, comprising the complex of formula (IV) (CAS nr. 478945-46-9, also known as iron (1+),chloro[dimethyl-9,9-dihydroxy-3-methyl-2,4-di-(2-pyridyl-kN)-7-[(2-pyridinyl-kN)methyl]-3,7-diazabicyclo[3.3.1]nonane-1,5-dicarboxylate-kN3,kN7]-,chloride(1-)).
- Nuodex DryCoat: contains a manganese-amine complex, equivalent to 1 wt% Mn, commercially available from Rockwood Pigments, UK (product number DCOAT).

According to the manufacturer, Nuodex Drycoat comprises <5% 2-ethyl-hexanol (CAS: 104-76-7, EG: 203-234-3), <10% Drycoat - Manganese complex (CAS: 1381939-25-8, EG: 937-913-7, registration number: 01-2119919049-35-0000), and 70-90% de-aromatised kerosene (CAS: 64742-48-9, EG: 265-150-3, registration number: 01-2119457273-39).
- Patcom 2516: contains a mixture of manganese 2-ethylhexanoate and 2,2'-bipyridyl, equivalent to 2.4 to 4.8 wt% of Mn, commercially available from Patcham. Patcom 2516 contains 15 - 30% of Mn 2-ethylhexanoate (EC 240-085-3, CAS 15956-58-8), 2.5-10% 2,2'-bipyridyl (EC 206-674-4, CAS 366-18-7), and 50-80% 2-butoxyethanol (EC 203-905-0, CAS 111-76-2).
- DriCAT 2700F: contains a complex comprising manganese octoate and a porphyrin ligand, equivalent to 0.8 wt% Mn, commercially available from Dura. DriCat 2700F comprises approximately 5 wt% of manganese carboxylate (EINECS 240-085-3, CAS 15956-58-8), approximately 60 wt% 1-hexanol, 2-ethyl- (EINECS 203-234-3, CAS 104-76-7), approximately 50 wt% distillates, petroleum, hydrotreated light (EINECS 265-149-8, CAS 64742-47-8).

- WorléeAdd VP 2500: contains a mixture of manganese 2-ethylhexanoate and tolyldiethanolamine, equivalent to 0,6 - 1.1 wt% Mn, commercially available from Worlée.
- Potassium Hex-Cem: contains potassium 2-ethylhexanoate, equivalent to 15 wt% K, commercially available from OMG

Drying times (dust-free and tack-free) are shown in hours:minutes. The time for development of a skin is shown in days.

The data in table 2 demonstrate that the coating composition and the drier composition according to embodiments of this invention show reduced skinning while maintaining excellent drying properties (even under adverse drying conditions such as low temperature and high humidity).

The metal concentrations of the examples in Table 2 and 3 are 0.0038% - 0.013% Mn (based on solids binder) and 0.00023% - 0.00116% Fe (based on solids binder).

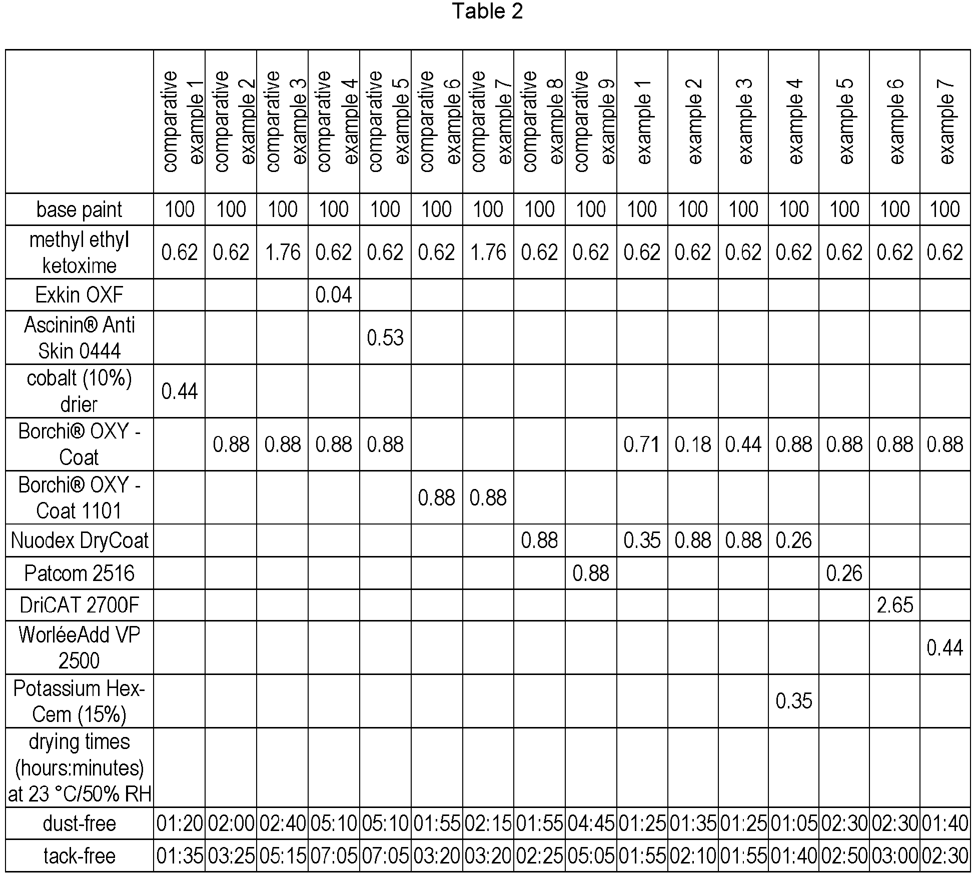

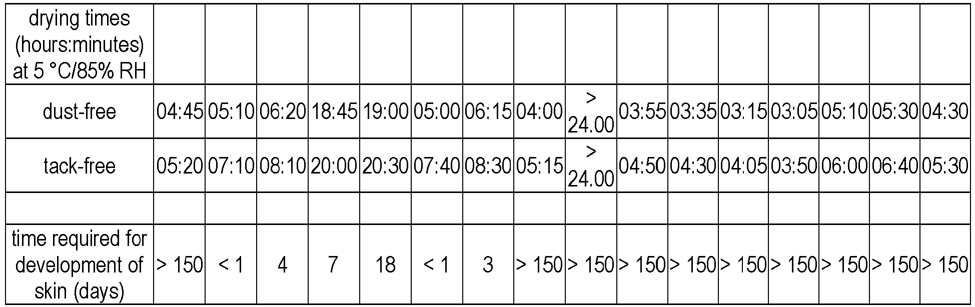

A white formulation was prepared by mixing together the constituents listed in Table 3.

Drying times (dust-free and tack-free) are shown in hours:minutes. The time for development of a skin is shown in days.

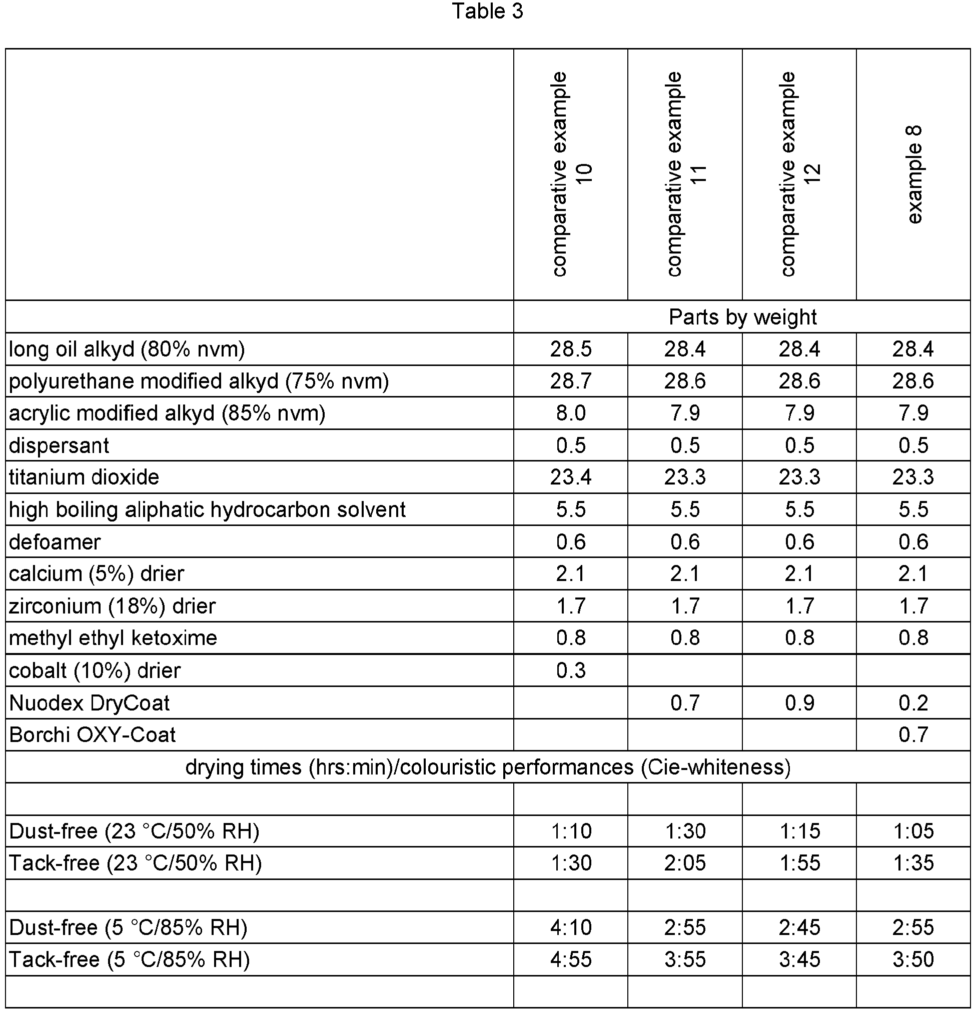

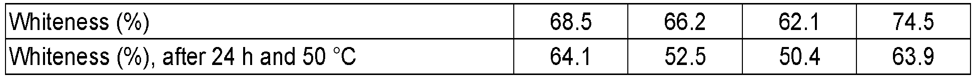

The data in table 3 demonstrate that in a white formulation (comparative examples 11 and 12), it is shown that high amounts of the Mn-based driers are required to obtain fast drying but these high Mn-concentrations compromise unacceptably the esthetical properties (whiteness and yellowing) of the film. It has been discovered that only low concentrations of Mn ions are needed for a successful performance of the composition 8 and that the level of discoloration of the dried coat of paint is at least tolerable and can even be lower than the levels obtained by conventional cobalt systems.

## Claims

1. A drier composition for an autoxidizable alkyd based coating composition, comprising:
a) at least one Fe complex comprising Fe and at least one nitrogen donor ligand, wherein said at least nitrogen donor ligand is selected from the group comprising tridentate, tetradentate, pentadentate and hexadentate nitrogen donor ligands;
b) at least one metal salt of a carboxylic acid, wherein the metal is selected from the group comprising: Mn, Ce, V, and Cu; and
c) at least one ligand comprising at least one moiety selected from the group comprising 1,4,7-tri-azacyclononanyl, 2,2'-bipyridyl, 1,10-phenantrolinyl, imidazolyl, pyrazolyl, porphyrinyl, aliphatic, cycloaliphatic, and aromatic amines.

2. A drier composition according to claim 1, wherein the ligand in a) is a compound of formula (I); 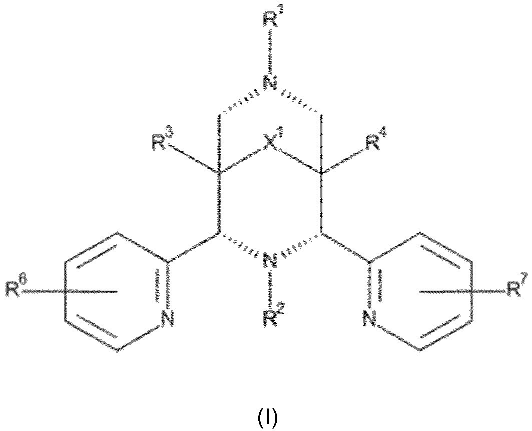
wherein R¹ is selected from the group consisting of C₁₋₂₄alkyl, C₆₋₁₀aryl, heteroaryl, heteroarylC₁₋₆alkyl, and C₁₋₆alkylaminoC₁₋₆alkyl, wherein heteroaryl is selected from the group consisting of pyridyl, pyrazinyl, pyrazolyl, pyrrolyl, imidazolyl, benzimidazolyl, pyrimidinyl, triazolyl and thiazolyl, preferably pyriddin-2yl; wherein R¹ is optionally substituted with one or more substituents, each independently selected from the group consisting of C₁₋₁₂alkyl, C₆₋₁₀aryl, C₆₋₁₀arylC₁₋₆alkyl;
R² is selected from the group consisting of C₁₋₂₄alkyl, C₆₋₁₀aryl, heteroaryl, heteroarylC₁₋₆alkyl, and C₁₋₆alkylaminoC₁₋₆alkyl, wherein heteroaryl is selected from the group consisting of pyridyl, pyrazinyl, pyrazolyl, pyrrolyl, imidazolyl, benzimidazolyl, pyrimidinyl, triazolyl and thiazolyl, preferably pyriddin-2yl; wherein R² is optionally substituted with one or more substituents, each independently selected from the group consisting of C₁₋₁₂alkyl, C₆₋₁₀aryl, C₆₋₁₀arylC₁₋₆alkyl;
R³ is selected from the group consisting of-H, C₁₋₁₂alkyl, C₁₋₁₂alkyl-O-C₁₋₁₂alkyl, C₁₋₁₂alkyl-O-C₆₋₁₀aryl, C₆₋₁₀aryl, hydroxyC₁₋₁₂alkyl, and -(CH₂)ₘC(O)OR⁵; wherein R⁵ is selected from -H, C₁₋₄alkyl, C₆₋₁₀aryl, or C₁₋₄alkylC₆₋₁₀aryl, and wherein m is an integer selected from 0, 1, 2, 3, or 4; wherein R³ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy;
R⁴ is selected from the group consisting of -H, C₁₋₁₂alkyl, C₁₋₁₂alkyl-O-C₁₋₁₂alkyl, C₁₋₁₂alkyl-O-C₆₋₁₀aryl, C₆₋₁₀aryl, hydroxyC₁₋₁₂alkyl, and -(CH₂)ₘC(O)OR⁵⁰; wherein R⁵⁰ is selected from -H, C₁₋₄alkyl, C₆₋₁₀aryl, or C₁₋₄alkylC₆₋₁₀aryl, and wherein m is an integer selected from 0, 1, 2, 3, or 4; wherein R⁴ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy;
R⁶ is selected from the group consisting of-H, halogen, -OH, C₁₋₆alkoxy, -NH-C(O)-H, -NH-C(O)-C₁₋₁₂alkyl, -NH₂, -NH-C₁₋₁₂alkyl, and C₁₋₁₂alkyl; wherein R⁶ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy;
R⁷ is selected from the group consisting of-H, halogen, -OH, C₁₋₆alkoxy, -NH-C(O)-H, -NH-C(O)-C₁₋₁₂alkyl, -NH₂, -NH-C₁₋₁₂alkyl, and C₁₋₁₂alkyl; wherein R⁷ is optionally substituted with one or more substituents, each independently selected from the group consisting of C₁₋₆alkyl, C₆₋₁₀aryl, halogen, C₁₋₆alkoxy, haloC₁₋₆alkyl, and haloC₁₋₆alkoxy;
X¹ is selected from -C(O)- or -[C(R⁸)₂]ₙ-; wherein n is an integer selected from 0, 1, 2 or 3, and each R⁸ is independently selected from the group consisting of -H, -OH, C₁₋₁₂alkoxy and C₁₋₁₂alkyl; wherein R⁸ is optionally substituted with one or more substituents, each independently selected from the group consisting of C₁₋₆alkyl, C₆₋₆aryl, halogen, C₁₋₆alkoxy, haloC₁₋₆alkyl, and haloC₁₋₆alkoxy.

3. A drier composition according to claim 1 or 2, wherein the ligand in a) is a compound of formula (I); 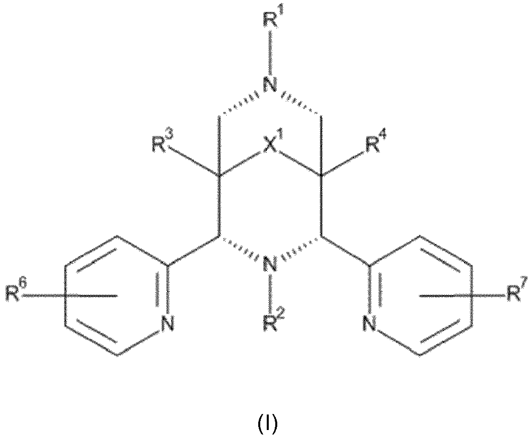
wherein R¹ and R² are each independently selected from the group consisting of C₁₋₂₄alkyl, C₆₋₁₀aryl, heteroaryl, heteroarylC₁₋₆alkyl, and C₁₋₆alkylaminoC₁₋₆alkyl; wherein heteroaryl is selected from the group consisting of pyridyl, pyrazinyl, and pyrazolyl ;
R³ and R⁴ are each independently selected from the group consisting of -H, C₁₋₈alkyl, C₁₋₈alkyl-O-C₁₋₈alkyl, C₁₋₈alkyl-O-C₆₋₁₀aryl, C₆₋₁₀aryl, hydroxyC₁₋₈alkyl, and -(CH₂)ₘC(O)OR⁵; R⁵ is selected from -H, C₁₋₄alkyl, C₆₋₁₀aryl, or C₁₋₄alkylC₆₋₁₀aryl, and m is an integer selected from 0, 1, 2, 3, or 4;
each R⁶ and R⁷ are independently selected from the group consisting of-H, -F, -CI, -Br, -OH, C₁₋₄alkoxy, -NH-C(O)-H, -NH-C(O)-C₁₋₄alkyl, -NH₂, -NH-C₁₋₄alkyl, and C₁₋₄alkyl; and,
X¹ is selected from -C(O)- or -[C(R⁸)₂]ₙ-; wherein n is an integer selected from 0, 1, 2 or 3, and each R⁸ is independently selected from the group consisting of-H, -OH, C₁₋₄alkoxy and C₁₋₄alkyl;

4. The drier composition according to any one of claims 1 to 3, further comprising:
d) a K salt of a carboxylic acid.

5. The drier composition according to any one of claims 1 to 4, wherein the metal in b) is selected from Mn or Ce, preferably, wherein the metal in b) is Mn.

6. The drier composition according to any one of claims 1 to 5, wherein the carboxylic acid is selected from branched-chain or straight-chain saturated and unsaturated aliphatic, aromatic and alicyclic monocarboxylic acids having 5 to 22 carbon atoms, cycloaliphatic monocarboxylic acids having 5 to 10 carbon atoms, or mixtures of these acids, preferably the carboxylic acid is selected from the group comprising 2-ethylbutanoic acid, 2,2-dimethylpentanoic acid, 2-ethylpentanoic acid, 2-ethyl-4-methylpentanoic acid, 2-ethylhexanoic acid, isooctanoic acid, isononanoic acid, neononanoic acid, nonanoic acid, isodecanoic acid, neodecanoic acid, 2-ethyldecanoic acid, isotridecanoic acid, isotetradecanoic acid, n-hexanoic acid, n-octanoic acid, n-decanoic acid, n-dodecanoic acid, cyclopentanoic acid, methylcyclopentanoic acid, cyclohexanoic acid, methylcyclohexanoic acid, 1,2-dimethylcyclohexanoic acid, cycloheptanoic acid, myristic acid, stearic acid, arachidic acid, behenic acid, oleic acid, linoleic acid, tall oil fatty acid, erucic acid, p-tert-butylbenzoic acid, monobutyl maleate, monodecyl phthalate, naphthenic acid and mixtures thereof.

7. The drier composition according to any one of claims 1 to 6, wherein the at least one ligand in c) is a compound of formula (V), wherein 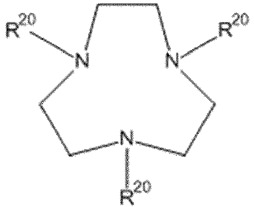 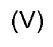
each R²⁰ is independently selected from the group consisting of C₁₋₆alkyl, C₃₋₈cycloalkyl, heterocycloalkyl, heteroaryl, C₆₋₁₀aryl and C₆₋₁₀aryl-C₁₋₆alkyl, each group being optionally substituted with one or more substituents each independently selected from the group consisting of -OH, C₁₋₆alkoxy, phenoxy, carboxylate, carboxamide, carboxylic ester, sulfonate, amine, C₁₋₆alkylamine and -N⁺(R²¹)₃;
each R²¹ is selected from -H, C₁₋₆alkyl, C₂₋₆alkenyl, C₆₋₁₀aryl-C₁₋₆alkyl, C₆₋₁₀aryl-C₂₋₆alkenyl, C₁₋₆alkyloxy, C₂₋₆alkenyloxy, aminoC₁₋₆alkyl, aminoC₂₋₆alkenyl, C₂₋₆alkyl ether, C₃₋₆alkenyl ether, and -CX²₂-R²²;
each X² is independently selected from -H or C₁₋₃alkyl and wherein each R²² is independently selected from an optionally substituted heteroaryl group selected from the group consisting of pyridyl, pyrazinyl, pyrazolyl, pyrrolyl, imidazolyl, benzimidazolyl, pyrimidinyl, triazolyl and thiazolyl; and,
wherein at least one of R²¹ is -C(X²)₂-R²², preferably wherein the organic compound in c) is 1,4,7-trimethyl-1,4,7-tri-azacyclononane.

8. The drier composition according to any one of claims 1 to 7, wherein components (b) and (c) of the drier composition are provided as the complex defined by CAS 1381939-25-8.

9. A coating composition, comprising:
a) at least one autoxidizable alkyd based binder; and
b) a drier composition according to any one of claims 1 to 8.

10. The coating composition according to claim 9, further comprising an oxime, preferably a ketoxime, preferably methyl ethyl ketoxime.

11. The coating composition according to claim 9 or 10, comprising at most 0.01 % by weight of antiskinning agent other than an oxime, preferably at most 0.001% by weight, preferably at most 0.0001% by weight, based on the total weight of the coating composition, preferably wherein the coating composition is essentially free of anti-skinning agents other than an oxime.

12. The coating composition according to any one of claims 9 to 11, wherein the coating composition comprises at least 0.0001 % to at most 2 % by weight of the metal in b), preferably at least 0.0002 % to at most 0.1 % by weight of the metal in b), more preferably at least 0.0005 % to at most 0.05 % by weight of the metal in b), for example at least 0.001 % to at most 0.02 % by weight of the metal in b), considering only the amount of metal of said at least one metal salt of a carboxylic acid, with weight percent being based on the total weight of the alkyd binder.

13. The coating composition according to any one of claims 9 to 12, wherein the coating composition is a solvent-borne coating composition.

14. Use of the coating composition according to any one of claims 9 to 13 in a varnish, lacquer, paint, stain, enamel, printing ink or floor covering.

15. A substrate having applied thereon a coating composition according to any one of claims 9 to 13.

## Patentansprüche

1. Trockenmittelzusammensetzung für eine selbstoxidierbare Beschichtungszusammensetzung auf Alkydbasis, umfassend:
a) wenigstens einen Fe-Komplex, der Fe und wenigstens einen Stickstoffdonatorliganden umfasst, wobei der wenigstens einen Stickstoffdonatorligand ausgewählt ist aus der Gruppe umfassend tridentate, tetradentate, pentadentate und hexadentate Stickstoffdonatorliganden;
b) wenigstens ein Metallsalz einer Carbonsäure, wobei das Metall ausgewählt ist aus der Gruppe umfassend: Mn, Ce, V und Cu; und
c) wenigstens einen Liganden umfassend wenigstens eine Einheit ausgewählt aus der Gruppe umfassend 1,4,7-Triazacyclononanyl, 2,2'-Bipyridyl, 1,10-Phenantrolinyl, Imidazolyl, Pyrazolyl, Porphyrinyl, aliphatische, cycloaliphatische und aromatische Amine.

2. Trockenmittelzusammensetzung gemäß Anspruch 1, wobei der Ligand in a) eine Verbindung der Formel (I) ist: 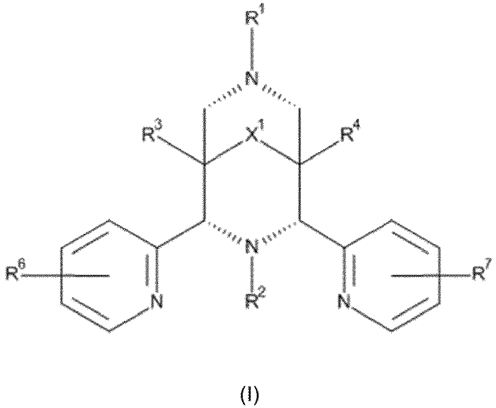
wobei R¹ ausgewählt ist aus der Gruppe bestehend aus C₁₋₂₄Alkyl, C₆₋₁₀Aryl, Heteroaryl, HeteroarylC₁₋₆alkyl und C₁₋₆AlkylaminoC₁₋₆alkyl, wobei Heteroaryl ausgewählt ist aus der Gruppe bestehend aus Pyridyl, Pyrazinyl, Pyrazolyl, Pyrrolyl, Imidazolyl, Benzimidazolyl, Pyrimidinyl, Triazolyl und Thiazolyl, vorzugsweise Pyridin-2-yl; wobei R¹ gegebenenfalls substituiert ist mit einem oder mehreren Substituenten, jeweils unabhängig ausgewählt aus der Gruppe bestehend aus C₁₋₁₂Alkyl, C₆₋₁₀Aryl, C₆₋₁₀ArylC₁₋₆alkyl;
R² ausgewählt ist aus der Gruppe bestehend aus C₁₋₂₄Alkyl, C₆₋₁₀Aryl, Heteroaryl, HeteroarylC₁₋₆alkyl und C₁₋₆AlkylaminoC₁₋₆alkyl, wobei Heteroaryl ausgewählt ist aus der Gruppe bestehend aus Pyridyl, Pyrazinyl, Pyrazolyl, Pyrrolyl, Imidazolyl, Benzimidazolyl, Pyrimidinyl, Triazolyl und Thiazolyl, vorzugsweise Pyridin-2-yl; wobei R² gegebenenfalls substituiert ist mit einem oder mehreren Substituenten, jeweils unabhängig ausgewählt aus der Gruppe bestehend aus C₁₋₁₂Alkyl, C₆₋₁₀Aryl, C₆₋₁₀ArylC₁₋₆alkyl;
R³ ausgewählt ist aus der Gruppe bestehend aus -H, C₁₋₁₂Alkyl, C₁₋₁₂Alkyl-O-C₁₋₁₂alkyl, C₁₋₁₂Alkyl-O-C₆₋₁₀aryl, C₆₋₁₀Aryl, HydroxyC₁₋₁₂alkyl und -(CH₂)ₘC(O)OR⁵; wobei R⁵ ausgewählt ist aus -H, C₁₋₄Alkyl, C₆₋₁₀Aryl oder C₁₋₄AlkylC₆₋₁₀aryl und wobei m eine ganze Zahl ausgewählt aus 0, 1, 2, 3 oder 4 ist; wobei R³ gegebenenfalls substituiert ist mit einem oder mehreren Substituenten, jeweils unabhängig ausgewählt aus der Gruppe bestehend aus C₁₋₆Alkyl, C₆₋₁₀Aryl, Halogen, C₁₋₆Alkoxy, HalogenC₁₋₆alkyl und HalogenC₁₋₆alkoxy;
R⁴ ausgewählt ist aus der Gruppe bestehend aus -H, C₁₋₁₂Alkyl, C₁₋₁₂Alkyl-O-C₁₋₁₂alkyl, C₁₋₁₂Alkyl-O-C₆₋₁₀aryl, C₆₋₁₀Aryl, HydroxyC₁₋₁₂alkyl und -(CH₂)ₘC(O)OR⁵⁰; wobei R⁵⁰ ausgewählt ist aus -H, C₁₋₄Alkyl, C₆₋₁₀Aryl oder C₁₋₄AlkylC₆₋₁₀aryl und wobei m eine ganze Zahl ausgewählt aus 0, 1, 2, 3 oder 4 ist; wobei R⁴ gegebenenfalls substituiert ist mit einem oder mehreren Substituenten, jeweils unabhängig ausgewählt aus der Gruppe bestehend aus C₁₋₆Alkyl, C₆₋₁₀Aryl, Halogen, C₁₋₆Alkoxy, HalogenC₁₋₆alkyl und HalogenC₁₋₆alkoxy;
R⁶ ausgewählt ist aus der Gruppe bestehend aus -H, Halogen, -OH, C₁₋₆Alkoxy, -NH-C(O)-H, -NH-C(O)-C₁₋₁₂Alkyl, -NH₂, -NH-C₁₋₁₂Alkyl und C₁₋₁₂Alkyl; wobei R⁶ gegebenenfalls substituiert ist mit einem oder mehreren Substituenten, jeweils unabhängig ausgewählt aus der Gruppe bestehend aus C₁₋₆Alkyl, C₆₋₁₀Aryl, Halogen, C₁₋₆Alkoxy, HalogenC₁₋₆alkyl und HalogenC₁₋₆alkoxy;
R⁷ ausgewählt ist aus der Gruppe bestehend aus -H, Halogen, -OH, C₁₋₆Alkoxy, -NH-C(O)-H, -NH-C(O)-C₁₋₁₂Alkyl, -NH₂, -NH-C₁₋₁₂Alkyl und C₁₋₁₂Alkyl; wobei R⁷ gegebenenfalls substituiert ist mit einem oder mehreren Substituenten, jeweils unabhängig ausgewählt aus der Gruppe bestehend aus C₁₋₆Alkyl, C₆₋₁₀Aryl, Halogen, C₁₋₆Alkoxy, HalogenC₁₋₆alkyl und HalogenC₁₋₆alkoxy;
X¹ ausgewählt ist aus -C(O)- oder -[C(R⁸)₂]ₙ-; wobei n eine ganze Zahl ausgewählt aus 0, 1, 2 oder 3 ist und jedes R⁸ unabhängig ausgewählt ist aus der Gruppe bestehend aus -H, -OH, C₁₋₁₂Alkoxy und C₁₋₁₂Alkyl; wobei R⁸ gegebenenfalls substituiert ist mit einem oder mehreren Substituenten, jeweils unabhängig ausgewählt aus der Gruppe bestehend aus C₁₋₆Alkyl, C₆₋₆Aryl, Halogen, C₁₋₆Alkoxy, HalogenC₁₋₆alkyl und HalogenC₁₋₆alkoxy.

3. Trockenmittelzusammensetzung gemäß Anspruch 1 oder 2, wobei der Ligand in a) eine Verbindung der Formel (I) ist: 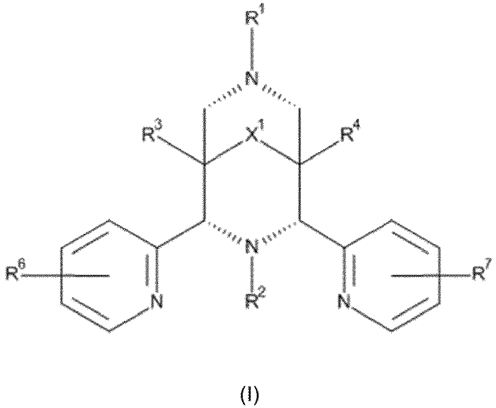
wobei R¹ und R² jeweils unabhängig ausgewählt sind aus der Gruppe bestehend aus C₁₋₂₄Alkyl, C₆₋₁₀Aryl, Heteroaryl, HeteroarylC₁₋₆alkyl und C₁₋₆AlkylaminoC₁₋₆alkyl; wobei Heteroaryl ausgewählt ist aus der Gruppe bestehend aus Pyridyl, Pyrazinyl und Pyrazolyl;
R³ und R⁴ jeweils unabhängig ausgewählt sind aus der Gruppe bestehend aus -H, C₁₋₈Alkyl, C₁₋₈Alkyl-O-C₁₋₈alkyl, C₁₋₈Alkyl-O-C₆₋₁₀aryl, C₆₋₁₀Aryl, HydroxyC₁₋₈alkyl und -(CH₂)ₘC(O)OR⁵; wobei R⁵ ausgewählt ist aus -H, C₁₋₄Alkyl, C₆₋₁₀Aryl oder C₁₋₄AlkylC₆₋₁₀aryl und wobei *m* eine ganze Zahl ausgewählt aus 0, 1, 2, 3 oder 4 ist;
jedes R⁶ und R⁷ unabhängig ausgewählt ist aus der Gruppe bestehend aus -H , -F, -Cl, -Br, -OH, C₁₋₄Alkoxy, -NH-C(O)-H, -NH-C(O)-C₁₋₄Alkyl, -NH₂, -NH-C₁₋₄Alkyl und C₁₋₄alkyl; und
X¹ ausgewählt ist aus -C(O)- oder -[C(R⁸)₂]ₙ-; wobei n eine ganze Zahl ausgewählt aus 0, 1, 2 oder 3 ist und jedes R⁸ unabhängig ausgewählt ist aus der Gruppe bestehend aus -H, -OH, C₁₋₄Alkoxy und C₁₋₄Alkyl.

4. Trockenmittelzusammensetzung gemäß einem der Ansprüche 1 bis 3, ferner umfassend:
d) ein K-Salz einer Carbonsäure.

5. Trockenmittelzusammensetzung gemäß einem der Ansprüche 1 bis 4, wobei das Metall in b) ausgewählt ist aus Mn oder Ce, wobei das Metall in b) vorzugsweise Mn ist.

6. Trockenmittelzusammensetzung gemäß einem der Ansprüche 1 bis 5, wobei die Carbonsäure ausgewählt ist aus verzweigten oder geradkettigen, gesättigten und ungesättigten aliphatischen, aromatischen und alicyclischen Monocarbonsäuren mit 5 bis 22 Kohlenstoffatomen, cycloaliphatischen Monocarbonsäuren mit 5 bis 10 Kohlenstoffatomen oder Gemischen dieser Säuren, wobei die Carbonsäure vorzugsweise ausgewählt ist aus der Gruppe umfassend 2-Ethylbutansäure, 2,2-Dimethylpentansäure, 2-Ethylpentansäure, 2-Ethyl-4-methylpentansäure, 2-Ethylhexansäure, Isooctansäure, Isononansäure, Neononansäure, Nonansäure, Isodecansäure, Neodecansäure, 2-Ethyldecansäure, Isotridecansäure, Isotetradecansäure, n-Hexansäure, n-Octansäure, n-Decansäure, n-Dodecansäure, Cyclopentansäure, Methylcyclopentansäure, Cyclohexansäure, Methylcyclohexansäure, 1,2-Dimethylcyclohexansäure, Cycloheptansäure, Myristinsäure, Stearinsäure, Arachinsäure, Behensäure, Ölsäure, Linolsäure, Tallölfettsäure, Erucasäure, p-tert-Butylbenzoesäure, Monobutylmaleat, Monodecylphthalat, Naphthensäure und Gemischen davon.

7. Trockenmittelzusammensetzung gemäß einem der Ansprüche 1 bis 6, wobei der wenigstens eine Ligand in c) eine Verbindung der Formel (V) ist, wobei 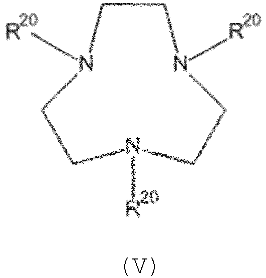
jedes R²⁰ unabhängig ausgewählt ist aus der Gruppe bestehend aus C₁₋₆Alkyl, C₃₋₈Cycloalkyl, Heterocycloalkyl, Heteroaryl, C₆₋₁₀Aryl und C₆₋₁₀ArylC₁₋₆alkyl, wobei jede Gruppe gegebenenfalls substituiert ist mit einem oder mehreren Substituenten, jeweils unabhängig ausgewählt aus der Gruppe bestehend aus -OH, C₁₋₆Alkoxy, Phenoxy, Carboxylat, Carboxamid, Carbonsäureester, Sulfonat, Amin, C₁₋₆Alkylamin und -N⁺(R²¹)₃;
jedes R²¹ ausgewählt ist aus -H, C₁₋₆-Alkyl, C_{2- 6}Alkenyl, C₆₋₁₀ArylC₁₋₆alkyl, C₆₋₁₀ArylC₂₋₆alkenyl, C_{1- 6}Alkyloxy, C₂₋₆Alkenyloxy, AminoC₁₋₆alkyl, AminoC_{2- 6}alkenyl, C₂₋₆Alkylether, C₃₋₆Alkenylether und -CX²₂-R²²;
jedes X² unabhängig ausgewählt ist aus -H oder C_{1- 3}Alkyl und wobei jedes R²² unabhängig ausgewählt ist aus einer gegebenenfalls substituierten Heteroarylgruppe ausgewählt aus der Gruppe bestehend aus Pyridyl, Pyrazinyl, Pyrazolyl, Pyrrolyl, Imidazolyl, Benzimidazolyl, Pyrimidinyl, Triazolyl und Thiazolyl; und
wobei wenigstens eines von R²¹ -C(X²)₂-R²² ist, wobei die organische Verbindung in c) vorzugsweise 1,4,7-Trimethyl-1,4,7-triazacyclononan ist.

8. Trockenmittelzusammensetzung gemäß einem der Ansprüche 1 bis 7, wobei die Komponenten (b) und (c) der Trockenmittelzusammensetzung als der von CAS 1381939-25-8 definierte Komplex bereitgestellt sind.

9. Beschichtungszusammensetzung, umfassend:
a) wenigstens ein selbstoxidierbares Bindemittel auf Alkydbasis; und
b) eine Trockenmittelzusammensetzung gemäß einem der Ansprüche 1 bis 8.

10. Beschichtungszusammensetzung gemäß Anspruch 9, ferner umfassend ein Oxim, vorzugsweise ein Ketoxim, vorzugsweise Methylethylketoxim.

11. Beschichtungszusammensetzung gemäß Anspruch 9 oder 10, umfassend höchstens 0,01 Gew.-%, vorzugsweise höchstens 0,001 Gew.-%, vorzugsweise höchstens 0,0001 Gew.-%, bezogen auf das Gesamtgewicht der Beschichtungszusammensetzung, Antihautbildungsmittel der von Oxim verschieden ist, wobei die Beschichtungszusammensetzung vorzugsweise im Wesentlichen frei von Antihautbildungsmitteln die von Oxim verschieden sind, ist.

12. Beschichtungszusammensetzung gemäß einem der Ansprüche 9 bis 11, wobei die Beschichtungszusammensetzung wenigstens 0,0001 Gew.-% bis höchstens 2 Gew.-% an dem Metall in b), vorzugsweise wenigstens 0,0002 Gew.-% bis höchstens 0,1 Gew.-% an dem Metall in b), bevorzugter wenigstens 0,0005 Gew.-% bis höchstens 0,05 Gew.-% an dem Metall in b), beispielsweise wenigstens 0,001 Gew.-% bis höchstens 0,02 Gew.-% an dem Metall in b), umfasst, wobei nur die Menge an Metall des wenigstens einen Metallsalzes einer Carbonsäure betrachtet wird, wobei die Gewichtsprozent auf das Gesamtgewicht des Alkyd-Bindemittels bezogen sind.

13. Beschichtungszusammensetzung gemäß einem der Ansprüche 9 bis 12, wobei die Beschichtungszusammensetzung eine Beschichtungszusammensetzung auf Lösungsmittelbasis ist.

14. Verwendung der Beschichtungszusammensetzung gemäß einem der Ansprüche 9 bis 13 in einem/einer Firnis, Lack, Anstrich, Lasur, Emaille, Druckfarbe oder Fußbodenbelag.

15. Substrat mit einer darauf aufgebrachten Beschichtungszusammensetzung gemäß einem der Ansprüche 9 bis 13.

## Revendications

1. Composition de siccatif pour une composition de revêtement à base d'alkyde auto-oxydable, comprenant :
a) au moins un complexe de Fe comprenant Fe et au moins un ligand donneur d'azote, ledit au moins un ligand donneur d'azote étant sélectionné dans le groupe comprenant des ligands donneurs d'azote tridentés, tétradentés, pentadentés et hexadentés ;
b) au moins un sel métallique d'un acide carboxylique, le métal étant sélectionné dans le groupe comprenant : Mn, Ce, V et Cu ; et
c) au moins un ligand comprenant au moins une fraction sélectionnée dans le groupe comprenant 1,4,7-triazacyclononanyle, 2,2'-bipyridyle, 1,10-phénantrolinyle, imidazolyle, pyrazolyle, porphyrinyle, aliphatiques, cycloaliphatiques, et amines aromatiques.

2. Composition de siccatif selon la revendication 1, dans laquelle le ligand dans a) est un composé ayant la formule (I) ; 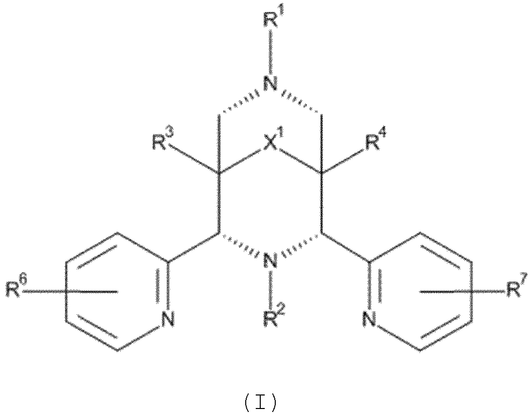
dans laquelle R¹ est sélectionné dans le groupe constitué d'alkyle C₁₋₂₄, aryle C₆₋₁₀, hétéroaryle, hétéroaryle-alkyle C₁₋₆, et alkylamino C₁₋₆-alkyle C₁₋₆, le hétéroaryle étant sélectionné dans le groupe constitué de pyridyle, pyrazinyle, pyrazolyle, pyrrolyle, imidazolyle, benzimidazolyle, pyrimidinyle, triazolyle et thiazolyle, préférablement pyridin-2-yle ; dans laquelle R¹ est optionnellement substitué par un ou plusieurs substituants, chacun sélectionné indépendamment dans le groupe constitué d'alkyle C₁₋₁₂, aryle C₆₋₁₀, aryle C₆₋₁₀-alkyle C₁₋₆ ;
dans laquelle R² est sélectionné dans le groupe constitué d'alkyle C₁₋₂₄, aryle C₆₋₁₀, hétéroaryle, hétéroaryle-alkyle C₁₋₆, et alkylamino C₁₋₆-alkyle C₁₋₆, le groupe hétéroaryle étant sélectionné dans le groupe constitué de pyridyle, pyrazinyle, pyrazolyle, pyrrolyle, imidazolyle, benzimidazolyle, pyrimidinyle, triazolyle et thiazolyle, préférablement pyridin-2-yle ; dans laquelle R² est optionnellement substitué par un ou plusieurs substituants, chacun sélectionné indépendamment dans le groupe constitué d' alkyle C₁₋₁₂, aryle C₆₋₁₀, aryle C₆₋₁₀-alkyle C₁₋₆ ;
R³ est sélectionné dans le groupe constitué de -H, alkyle C₁₋₁₂, alkyle C₁₋₁₂-O-alkyle C₁₋₁₂, alkyle C₁₋₁₂-O-aryle C₆₋₁₀, aryle C₆₋₁₀, hydroxyalkyle C₁₋₁₂ et -(CH₂)*ₘ*C(O)OR⁵ ; R⁵ étant sélectionné parmi -H, alkyle C₁₋₄, aryle C₆₋₁₀ ou alkyle C₁₋₄-aryle C₆₋₁₀, et dans lequel *m* est un nombre entier sélectionné parmi 0, 1, 2, 3 ou 4 ; R³ étant optionnellement substitué par un ou plusieurs substituants, chacun sélectionné indépendamment dans le groupe constitué d' alkyle C₁₋₆, aryle C₆₋₁₀, halogène, alcoxy C₁₋₆, haloalkyle C₁₋₆ et haloalcoxy C₁₋₆ ;
R⁴ est sélectionné dans le groupe constitué de -H, alkyle C₁₋₁₂, alkyle C₁₋₁₂-O-alkyle C₁₋₁₂, alkyle C₁₋₁₂-O-aryle C₆₋₁₀, aryle C₆₋₁₀, hydroxyalkyle C₁₋₁₂ et -(CH₂)*ₘ*C(O)OR⁵⁰ ; R⁵⁰ étant sélectionné parmi -H, alkyle C₁₋₄, aryle C₆₋₁₀ ou alkyle C₁₋₄-aryle C₆₋₁₀, et dans lequel *m* est un nombre entier sélectionné parmi 0, 1, 2, 3 ou 4 ; R⁴ étant optionnellement substitué par un ou plusieurs substituants, chacun sélectionné indépendamment dans le groupe constitué d' alkyle C₁₋₆, aryle C₆₋₁₀, halogène, alcoxy C₁₋₆, haloalkyle C₁₋₆ et haloalcoxy C₁₋₆ ;
R⁶ est sélectionné dans le groupe constitué de -H, halogène, -OH, alcoxy C₁₋₆, -NH-C(O)-H, -NH-C(O)-alkyle C₁₋₁₂, -NH₂, -NH-alkyle C₁₋₁₂ et alkyle C₁₋₁₂ ; R⁶ étant optionnellement substitué par un ou plusieurs substituants, chacun sélectionné indépendamment dans le groupe constitué d'alkyle C₁₋₆, aryle C₆₋₁₀, halogène, alcoxy C₁₋₆, haloalkyle C₁₋₆ et haloalcoxy C₁₋₆ ;
R⁷ est sélectionné dans le groupe constitué de -H, halogène, -OH, alcoxy C₁₋₆, -NH-C(O)-H, -NH-C(O)-alkyle C₁₋₁₂, -NH₂, -NH-alkyle C₁₋₁₂ et alkyle C₁₋₁₂ ; R⁷ étant optionnellement substitué par un ou plusieurs substituants, chacun sélectionné indépendamment dans le groupe constitué d'alkyle C₁₋₆, aryle C₆₋₁₀, halogène, alcoxy C₁₋₆, haloalkyle C₁₋₆ et haloalcoxy C₁₋₆ ;
X¹ est sélectionné parmi -C(O)- ou -[C(R⁸)₂]*ₙ*- ; n étant un nombre entier sélectionné parmi 0, 1, 2 ou 3, et chaque groupe R⁸ étant sélectionné indépendamment dans le groupe constitué de -H, -OH, alcoxy C₁₋₁₂ et alkyle C₁₋₁₂ ; R⁸ étant optionnellement substitué par un ou plusieurs substituants, chacun sélectionné indépendamment dans le groupe constitué d' alkyle C₁₋₆, aryle C₆₋₆, halogène, alcoxy C₁₋₆, haloalkyle C₁₋₆ et haloalcoxy C₁₋₆.

3. Composition de siccatif selon la revendication 1 ou 2, dans laquelle le ligand dans a) est un composé ayant la formule (I) ; 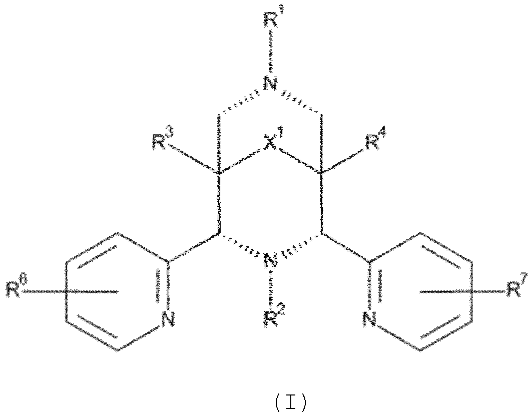
dans laquelle R¹ et R² sont chacun sélectionné indépendamment dans le groupe constitué d' alkyle C₁₋₂₄, aryle C₆₋₁₀, hétéroaryle, hétéroaryle-alkyle C₁₋₆, et alkylamino C₁₋₆-alkyle C₁₋₆, le groupe hétéroaryle étant sélectionné dans le groupe constitué de pyridyle, pyrazinyle, et pyrazolyle ;
R³ et R⁴ sont chacun sélectionné indépendamment dans le groupe constitué de -H, alkyle C₁₋₈, alkyle C₁₋₈-O-alkyle C₁₋₈, alkyle C₁₋₈-O-aryle C₆₋₁₀, aryle C₆₋₁₀, hydroxyalkyle C₁₋₈ et -(CH₂)*ₘ*C(O)OR⁵ ; R⁵ étant sélectionné parmi -H, alkyle C₁₋₄, aryle C₆₋₁₀ ou alkyle C₁₋₄-aryle C₆₋₁₀, et *m* étant un nombre entier sélectionné parmi 0, 1, 2, 3 ou 4 ;
chaque R⁶ et R⁷ est sélectionné indépendamment dans le groupe constitué de -H, -F, -Cl, -Br, -OH, alcoxy C₁₋₄, - NH-C(O)-H, -NH-C(O)-alkyle C₁₋₄, -NH₂, -NH-alkyle C₁₋₄, et alkyle C₁₋₄ ; et
X¹ est sélectionné parmi -C(O)- ou -[C(R⁸)₂]*ₙ*- ; dans lesquels n est un nombre entier sélectionné parmi 0, 1, 2 ou 3, et chaque R⁸ est sélectionné indépendamment dans le groupe constitué de -H, -OH, alcoxy C₁₋₄ et alkyle C₁₋₄.

4. Composition de siccatif selon l'une quelconque des revendications 1 à 3, comprenant en outre : d) un sel potassique d'un acide carboxylique.

5. Composition de siccatif selon l'une quelconque des revendications 1 à 4, dans laquelle le métal dans b) est sélectionné parmi Mn ou Ce, le métal dans b) étant préférablement Mn.

6. Composition de siccatif selon l'une quelconque des revendications 1 à 5, dans laquelle l'acide carboxylique est sélectionné parmi des acides monocarboxyliques aliphatiques, aromatiques et alicycliques saturés et insaturés à chaîne ramifiée ou à chaîne linéaire comportant 5 à 22 atomes de carbone, des acides monocarboxyliques cycloaliphatiques comportant 5 à 10 atomes de carbone, ou des mélanges de ces acides, l'acide carboxylique étant préférablement sélectionné dans le groupe comprenant l'acide 2-éthylbutanoïque, l'acide 2,2-diméthylpentanoïque, l'acide 2-éthylpentanoïque, l'acide 2-éthyl-4-méthylpentanoïque, l'acide 2-éthylhexanoïque, l'acide isooctanoïque, l'acide isononanoïque, l'acide néononanoïque, l'acide nonanoïque, l'acide isodécanoïque, l'acide néodécanoïque, l'acide 2-éthyldécanoïque, l'acide isotridécanoïque, l'acide isotétradécanoïque, l'acide n-hexanoïque, l'acide n-octanoïque, l'acide n-décanoique, l'acide n-dodécanoïque, l'acide cyclopentanoïque, acide méthylcyclopentanoïque, acide cyclohexanoïque, l'acide méthylcyclohexanoïque, l'acide 1,2-diméthylcyclohexanoïque, l'acide cycloheptanoïque, l'acide myristique, l'acide stéarique, l'acide arachidique, l'acide béhénique, l'acide oléique, l'acide linoléique, un acide gras de tallol, l'acide érucique, l'acide p-tert-butylbenzoïque, le maléate de monobutyle, le phtalate de monodécyle, l'acide naphténique, et des mélanges de ceux-ci.

7. Composition de siccatif selon l'une quelconque des revendications 1 à 6, dans laquelle l'au moins un ligand dans c) est un composé ayant la formule (V), dans laquelle 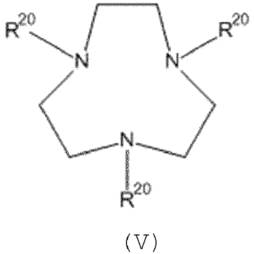
chaque groupe R²⁰ est sélectionné indépendamment dans le groupe constitué) d'alkyle C₁₋₆, cycloalkyle C₃₋₈, hétérocycloalkyle, hétéroaryle, aryle C₆₋₁₀ et aryle C₆₋₁₀-alkyle C₁₋₆, chaque groupe étant optionnellement substitué par un ou plusieurs substituants chacun sélectionné indépendamment dans le groupe constitué de -OH, alcoxy C₁₋₆, phénoxy, carboxylate, carboxamide, ester carboxylique, sulfonate, amine, alkylamine et - N⁺(R²¹)₃ ;
chaque groupe R²¹ est sélectionné parmi -H, alkyle C₁₋₆, alcényle C₂₋₆, aryle C₆₋₁₀-alkyle C₁₋₆, aryle C₆₋₁₀-alcényle C₂₋₆, alkyloxy C₁₋₆, alcényloxy C₂₋₆, aminoalkyle C₁₋₆, aminoalcényle C₂₋₆, éther alkylique C₂₋₆, éther alcénylique C₃₋₆, et -CX²₂-R²² ;
chaque X² est sélectionné indépendamment parmi -H ou alkyle C₁₋₃ et chaque R²² est sélectionné indépendamment parmi un groupe hétéroaryle optionnellement substitué sélectionné dans le groupe constitué de pyridyle, pyrazinyle, pyrazolyle, pyrrolyle, imidazolyle, benzimidazolyle, pyrimidinyle, triazolyle et thiazolyle ; et
dans laquelle au moins l'un des R²¹ est -C(X²)₂-R²², préférablement dans laquelle le composé organique dans c) est le 1,4,7-triméthyl-1,4,7-tri-azacyclonane.

8. Composition de siccatif selon l'une quelconque des revendications 1 à 7, dans laquelle les composants (b) et (c) de la composition de siccatif sont fournis sous la forme du complexe défini par CAS 1381939-25-8.

9. Composition de revêtement, comprenant :
a) au moins un liant à base d'alkyde auto-oxydable ; et
b) une composition de siccatif selon l'une quelconque des revendications 1 à 8.

10. Composition de revêtement selon la revendication 9, comprenant en outre une oxime, préférablement une cétoxime, préférablement une méthyléthylcétoxime.

11. Composition de revêtement selon la revendication 9 ou 10, comprenant au maximum 0,01 % en poids d'agent anti-peau autre qu'une oxime, préférablement au maximum 0,001 % en poids, préférablement au maximum 0,0001 % en poids, relativement au poids total de la composition de revêtement, la composition de revêtement étant préférablement essentiellement exempte d'agents anti-peau autres qu'une oxime.

12. Composition de revêtement selon l'une quelconque des revendications 9 à 11, la composition de revêtement comprenant au moins 0,0001 % à un maximum de 2 % en poids du métal dans b), préférablement au moins 0,0002 % à un maximum de 0,1 % en poids du métal dans b), plus préférablement au moins 0,0005 % à un maximum de 0,05 % en poids du métal dans b), par exemple au moins 0,001 % à un maximum de 0,02 % en poids du métal dans b), en tenant compte uniquement de la quantité de métal dudit au moins un sel métallique d'un acide carboxylique, le pourcentage en poids étant basé sur le poids total du liant alkyde.

13. Composition de revêtement selon l'une quelconque des revendications 9 à 12, la composition de revêtement étant une composition de revêtement à base de solvant.

14. Utilisation de la composition de revêtement selon l'une quelconque des revendications 9 à 13 dans un vernis, une laque, une peinture, une teinture, un émail, une encre d'impression ou un revêtement de sol.

15. Substrat sur lequel a été appliquée une composition de revêtement selon l'une quelconque des revendications 9 à 13.
